(19) Europäisches Patentamt
European Patent Office
Office européen des brevets

(11) **EP 3 159 353 A1**

(12) **EUROPEAN PATENT APPLICATION**
published in accordance with Art. 153(4) EPC

(43) Date of publication:
**26.04.2017 Bulletin 2017/17**

(21) Application number: **15810504.9**

(22) Date of filing: **13.02.2015**

(51) Int Cl.:
*C07K 5/12* (2006.01)     *A23G 1/00* (2006.01)
*A23G 1/30* (2006.01)     *A23L 2/00* (2006.01)
*C07D 241/08* (2006.01)     *C07K 5/06* (2006.01)

(86) International application number:
**PCT/JP2015/054015**

(87) International publication number:
**WO 2015/194205 (23.12.2015 Gazette 2015/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **20.06.2014   PCT/JP2014/066425**

(71) Applicant: **Suntory Holdings Limited**
**Osaka-shi, Osaka 530-8203 (JP)**

(72) Inventors:
• **SUZUKI, Toshihide**
**Kyoto 619-0284 (JP)**

• **FUKIZAWA, Shinya**
**Kyoto 619-0284 (JP)**
• **BEPPU, Yoshinori**
**Kyoto 619-0284 (JP)**
• **WATANABE, Hiroshi**
**Kyoto 619-0284 (JP)**

(74) Representative: **Vossius & Partner**
**Patentanwälte Rechtsanwälte mbB**
**Siebertstrasse 3**
**81675 München (DE)**

(54) **CYCLIC DIPEPTIDE-CONTAINING COMPOSITION**

(57)     A cyclic dipeptide-containing composition containing each of tyrosine-containing cyclic dipeptides selected from the group consisting of cyclotryptophanyltyrosine, cycloseryltyrosine, cycloprolyltyrosine, cyclotyrosylglycine, cyclotyrosyltyrosine, cyclophenylalanyltyrosine, cycloleucyltyrosine, cyclolysyltyrosine, cyclohistidyltyrosine, cycloalanyltyrosine, cycloglutamyltyrosine, cyclovalyltyrosine, cycloisoleucyltyrosine, cyclothreonyltyrosine, cycloaspartyltyrosine, cycloasparaginyltyrosine, cycloglutaminyltyrosine, cycloarginyltyrosine, and cyclomethionyltyrosine, or a salt thereof in a specified amount. The cyclic dipeptide-containing composition of the present invention has an excellent action of lowering a uric acid level, and the cyclic dipeptide-containing composition is useful in, for example, prevention or treatment of hyperuricemia, gout or the like.

[FIG. 4]

FIG.4

* p<0.05 (vs PO)

**EP 3 159 353 A1**

**Description**

TECHNICAL FIELD

The present invention relates to a cyclic dipeptide-containing composition.

BACKGROUND ART

"Dipeptides" in which two amino acids are bonded have been remarked as functional materials. The dipeptides make it possible to add a physical property not found in a single amino acid, or add a new function, so that the dipeptides have been expected to have a wider range of applications beyond the amino acids. Among them, diketopiperazines, which are cyclic dipeptides, have been known to have various physiological activities, and it has been expected that the diketopiperazines will expand in demands in the fields of medicine and pharmacology.

For example, Patent Publication 1 has reported that cyclic dipeptides having 2,5-diketopiperazine structures have anti-depressant action, action for improving learning motivation or the like. Also, Non-Patent Publication 1 describes that a cyclic dipeptide Cyclo(His-Pro) shows diversified physiological activities, including actions involving the central nervous system, such as lowering of body temperature and suppression of appetite; and hormone-like actions such as inhibition of prolactin secretion and promotion of growth hormone secretion, and the publication also reports that a cyclic dipeptide Cyclo(Leu-Gly) shows actions for improving memory functions, and that a cyclic dipeptide Cyclo(Asp-Pro) shows an action of inhibiting fat preference. Non-Patent Publication 2 has reported cyclic dipeptides having an anti-bacterial action and an antioxidative action.

In addition, Non-Patent Publication 3 describes that a cyclic dipeptide Cyclo(Trp-Pro) has an anti-cancer action, that cyclic dipeptides Cyclo(His-Pro) and Cyclo(Gly-Pro) have an anti-bacterial action, that a cyclic dipeptide Cyclo(His-Pro) has a neuroprotectant action, that a cyclic dipeptide Cyclo(Gly-Pro) has an action for improving memory functions, and that cyclic dipeptides Cyclo(Tyr-Pro) and Cyclo(Phe-Pro) have an action as a biological herbicide.

PRIOR ART REFERENCES

PATENT PUBLICATIONS

Patent Publication 1: Japanese Unexamined Patent Publication No. 2012-517998

NON-PATENT PUBLICATIONS

Non-Patent Publication 1: Peptides, 16(1), 151-164 (1995)
Non-Patent Publication 2: Biosciences and Industries, 60(7), 454-457 (2002)
Non-Patent Publication 3: Chemical Reviews, 112, 3641-3716 (2012)

SUMMARY OF THE INVENTION

PROBLEMS TO BE SOLVED BY THE INVENTION

However, there are many unclear functions for cyclic dipeptides.

An object of the present invention is to provide a composition containing a cyclic dipeptide having an excellent action of lowering a uric acid level.

MEANS TO SOLVE THE PROBLEMS

The present invention relates to the following [1] to [3]:

[1] a cyclic dipeptide-containing composition, containing one or more tyrosine-containing cyclic dipeptides or a salt thereof of the following (1) to (8) each in an amount satisfying ranges of:

(1) content of cyclolysyltyrosine or a salt thereof: $1.00 \times 10$ to $1.50 \times 10^5$ ppm,
(2) content of cycloisoleucyltyrosine or a salt thereof: $0.80 \times 10$ to $1.30 \times 10^5$ ppm,
(3) content of cyclothreonyltyrosine or a salt thereof: $0.50 \times 10$ to $0.90 \times 10^5$ ppm,

(4) content of cycloaspartyltyrosine or a salt thereof: $0.10 \times 10$ to $0.30 \times 10^5$ ppm,
(5) content of cycloasparaginyltyrosine or a salt thereof: $0.50 \times 10$ to $0.90 \times 10^5$ ppm,
(6) content of cycloglutaminyltyrosine or a salt thereof: $0.30 \times 10$ to $0.50 \times 10^5$ ppm,
(7) content of cycloarginyltyrosine or a salt thereof: $1.00 \times 10$ to $1.60 \times 10^5$ ppm, and
(8) content of cyclomethionyltyrosine or a salt thereof: $0.10 \times 10$ to $0.20 \times 10^5$ ppm;

[2] a cyclic dipeptide-containing composition, containing one or more tyrosine-containing cyclic dipeptides or a salt thereof of the following (1) to (8) each in an amount satisfying ranges of:

(1) content of cyclolysyltyrosine or a salt thereof: $5.0 \times 10^{-4}$ to $9.0 \times 10$ mg/100 mL,
(2) content of cycloisoleucyltyrosine or a salt thereof: $4.0 \times 10^{-4}$ to $7.0 \times 10$ mg/100 mL,
(3) content of cyclothreonyltyrosine or a salt thereof: $3.0 \times 10^{-4}$ to $5.0 \times 10$ mg/100 mL,
(4) content of cycloaspartyltyrosine or a salt thereof: $1.0 \times 10^{-4}$ to $2.0 \times 10$ mg/100 mL,
(5) content of cycloasparaginyltyrosine or a salt thereof: $3.0 \times 10^{-4}$ to $5.0 \times 10$ mg/100 mL,
(6) content of cycloglutaminyltyrosine or a salt thereof: $1.0 \times 10^{-4}$ to $3.0 \times 10$ mg/100 mL,
(7) content of cycloarginyltyrosine or a salt thereof: $6.0 \times 10^{-4}$ to $1.0 \times 10^2$ mg/100 mL, and
(8) content of cyclomethionyltyrosine or a salt thereof: $0.7 \times 10^{-4}$ to $2.0 \times 10$ mg/100 mL; and

[3] a cyclic dipeptide-containing composition, containing one or more tyrosine-containing cyclic dipeptides or a salt thereof of the following (1) to (8) each in an amount satisfying ranges of:

(1) content of cyclolysyltyrosine or a salt thereof: 0.01 to 15% by weight,
(2) content of cycloisoleucyltyrosine or a salt thereof: 0.008 to 13% by weight,
(3) content of cyclothreonyltyrosine or a salt thereof: 0.005 to 9% by weight,
(4) content of cycloaspartyltyrosine or a salt thereof: 0.001 to 3% by weight,
(5) content of cycloasparaginyltyrosine or a salt thereof: 0.005 to 9% by weight,
(6) content of cycloglutaminyltyrosine or a salt thereof: 0.003 to 5% by weight,
(7) content of cycloarginyltyrosine or a salt thereof: 0.01 to 16% by weight, and
(8) content of cyclomethionyltyrosine or a salt thereof: 0.001 to 2% by weight.

## EFFECTS OF THE INVENTION

The cyclic dipeptide-containing composition of the present invention exhibits an excellent effect of having an excellent action of lowering a uric acid level.

## BRIEF DESCRIPTION OF THE DRAWINGS

[FIG. 1] FIG. 1 is a diagram showing xanthine oxidase inhibition rates (%) of Cyclo(Tyr-Gly), linear dipeptides Tyr-Gly and Gly-Tyr, and an amino acid Tyr.
[FIG. 2] FIG. 2 is a diagram showing the results of studies on the actions of lowering serum uric acid levels of tyrosine-containing cyclic dipeptides for hyperuricemic mice.
[FIG. 3] FIG. 3 is a diagram showing the results of studies on actions of inhibiting xanthine oxidase (XO) activity of tyrosine-containing cyclic dipeptides for hyperuricemic mice, wherein the upper panel is a diagram showing XO activity in the liver, and the lower panel is a diagram showing XO activity in the sera.
[FIG. 4] FIG. 4 is a diagram showing the results of studies on actions of lowering serum uric acid levels of soybean peptide heat-treated product, and a mixture of 8 kinds of Tyr-containing cyclic dipeptides contained in the soybean peptide heat-treated product for hyperuricemic mice.
[FIG. 5] FIG. 5 is a diagram showing the results of studies on the actions of lowering serum uric acid levels depending upon the dosages of the soybean peptide heat-treated products for hyperuricemic mice.

## MODES FOR CARRYING OUT THE INVENTION

The cyclic dipeptide-containing composition of the present invention contains one or more cyclic dipeptides selected from the group consisting of cyclotryptophanyltyrosine [Cyclo(Trp-Tyr)], cycloseryltyrosine [Cyclo(Ser-Tyr)], cycloprolyltyrosine [Cyclo(Pro-Tyr)], cyclotyrosylglycine [Cyclo(Tyr-Gly)], cyclotyrosyltyrosine [Cyclo(Tyr-Tyr)], cyclophenylalanyltyrosine [Cyclo(Phe-Tyr)], cycloleucyltyrosine [Cyclo(Leu-Tyr)], cyclolysyltyrosine [Cyclo(Lys-Tyr)], cyclohistidyltyrosine [Cyclo(His-Tyr)], cycloalanyltyrosine [Cyclo(Ala-Tyr)], cycloglutamyltyrosine [Cyclo(Glu-Tyr)], cyclovalyltyrosine [Cyclo(Val-Tyr)], cycloisoleucyltyrosine [Cyclo(Ile-Tyr)], cyclothreonyltyrosine [Cyclo(Thr-Tyr)], cycloaspartyltyrosine [Cyclo(Asp-Tyr)], cycloasparaginyltyrosine [Cyclo(Asn-Tyr)], cycloglutaminyltyrosine [Cyclo(Gln-Tyr)], cycloarginyltyrosine [Cyclo(Arg-Tyr)], and cyclomethionyltyrosine [Cyclo(Met-Tyr)], or salts thereof, the cyclic dipeptide containing tyrosine, wherein the cyclic dipeptide has some features that the content of the cyclic dipeptide or a salt thereof is in a specified amount. Among them, it is preferable that compositions contain one or more cyclic dipeptides selected from the group consisting of cyclolysyltyrosine [Cyclo(Lys-Tyr)], cycloisoleucyltyrosine [Cyclo(Ile-Tyr)], cyclothreonyltyrosine [Cyclo(Thr-Tyr)], cycloaspartyltyrosine [Cyclo(Asp-Tyr)], cycloasparaginyltyrosine [Cyclo(Asn-Tyr)], cycloglutaminyltyrosine [Cyclo(Gln-Tyr)], cycloarginyltyrosine [Cyclo(Arg-Tyr)], and cyclomethionyltyrosine[Cyclo(Met-Tyr)], or salts thereof, wherein the content of the cyclic dipeptide or a salt thereof is a specified amount, from the viewpoint of solubility in water. The above tyrosine-containing cyclic dipeptide may be hereinafter also referred to as the cyclic dipeptide of the present invention or the diketopiperazine of the present invention. Here, in the present specification, so long as the constitution of the amino acids in the cyclic dipeptides is the same, it does not matter which amino acids are written first. For example, Cyclo(Trp-Tyr) and Cyclo(Tyr-Trp) are the same cyclic dipeptide.

The cyclic dipeptide of the present invention may be any one so long as at least one of the constituting amino acids is tyrosine. For example, in a case where the two constituting amino acids are named an amino acid A and an amino acid B, the cyclic dipeptide has a structure such that a carboxy group of the amino acid A and an amino group of the amino acid B may be subjected to dehydration condensation, and an amino group of the amino acid A and a carboxy group of the amino acid B are subjected to dehydration condensation. The cyclic dipeptide of the present invention as described above contains tyrosine as a constituent. Although the detailed reasons are not elucidated, in consideration of the matters that the phenylalanine-containing cyclic dipeptide having the same aromatic ring is not confirmed to have an action of inhibiting a xanthine oxidase according to the comparison made between Tables 1 and 3 given later, it is assumed that the activity of a xanthine oxidase is inhibited by a phenolic hydroxyl group of the tyrosine. However, these assumptions are not construed to limit the present invention.

The cyclic dipeptide-containing composition of the present invention contains a specified amount of one or more cyclic dipeptides among the nineteen cyclic dipeptides listed above or salts thereof. Among them, a composition containing at least one cyclic dipeptide selected from the group consisting of cyclolysyltyrosine, cycloisoleucyltyrosine, cyclothreonyltyrosine, cycloaspartyltyrosine, cycloasparaginyltyrosine, cycloglutaminyltyrosine, cycloarginyltyrosine, and cyclomethionyltyrosine, or salts thereof, and the content of the cyclic dipeptide or a salt thereof is a specified amount, from the viewpoint of solubility in water. These cyclic dipeptides having excellent solubility in water have a solubility in water at 25°C of 1 mM or more, and cyclolysyltyrosine, cycloaspartyltyrosine, cycloarginyltyrosine, and cyclomethionyltyrosine, having a solubility in water at 25°C of 5 mM or more are more preferred. The cyclic dipeptide or a salt thereof may be simply collectively referred to as the cyclic dipeptide.

The salts of the cyclic dipeptide as used herein refer to any optional pharmacologically acceptable salts of the above-mentioned cyclic dipeptides, including inorganic salts and organic salts, and the salts include, for example, sodium salts, potassium salts, calcium salts, magnesium salts, ammonium salts, hydrochlorides, sulfates, nitrates, phosphates, salts of organic acids (acetates, citrates, maleates, malates, oxalates, lactates, succinates, fumarates, propionates, formates, benzoates, picrates, benzenesulfonates, and the like), and the like of the above cyclic dipeptides.

The cyclic dipeptide used in the present invention can be prepared in accordance with a method known in the art. For example, the cyclic dipeptide may be produced by a chemical synthesis method, an enzyme method, or a microbial fermentation method, or the cyclic dipeptide may be synthesized by subjecting a linear peptide to dehydration and cyclization reaction, or the cyclic dipeptide can also be prepared in accordance with a method described in Japanese Patent Laid-Open No. 2003-252896 or J. Peptide Sci., 10, 737-737 (2004). In the present invention, a processed product obtained by subjecting a solution containing a soybean peptide to a heat treatment can be suitably used.

The processed product obtained by subjecting a solution containing a soybean peptide to a heat treatment refers specifically to one that can be prepared, for example, by dissolving a soybean peptide in water at a concentration of from 20 to 500 mg/mL, and heating the solution under the conditions of from 40° to 150°C for 5 minutes to 120 hours. The processed product obtained may be subjected to a treatment such as filtration, centrifugation, concentration, ultrafiltration, lyophilization, or powdering as desired.

The salt of the cyclic dipeptide can be easily prepared by one of ordinary skill in the art in accordance with any methods that are known in the art.

Since the composition of the present invention has the feature that the cyclic dipeptide or a salt thereof of the present invention is contained in a specified amount, the composition can inhibit the activity of a xanthine oxidase to control the generation of uric acid. Therefore, the composition can be suitably used for a disease in need of an action of lowering uric acid levels. Accordingly, as the composition of the present invention, compositions in which the above-mentioned cyclic dipeptides or salts thereof are blended in an amount defined in the present invention are embraced in the present invention.

The diseases that are in need of an action for lowering uric acid levels in the present invention are not particularly limited so long as the diseases are observed with some therapeutic effects by lowering a blood uric acid level. The diseases are exemplified by, for example, hyperuricemia, gout, gouty tophus, acute gouty arthritis, chronic gouty arthritis, gouty kidney, ureteral calculus, renal dysfunction, joint function disorder, vascular disorder, and the like.

In addition, the composition of the present invention is highly useful in amelioration or prevention of symptoms of a disease in need of actions of lowering uric acid levels for treatment or prevention by actions of inhibiting a xanthine oxidase owned by the cyclic dipeptides or salts thereof of the present invention. In other words, it is made possible to provide the composition of the present invention as foods with health claims or dietary supplements having the purposes of preventing or ameliorating the above diseases, with the indications that, for example, the composition can be used for the prevention and/or amelioration of gout/hyperuricemia, which would serve as very useful compositions for individuals having somewhat higher blood uric acid levels, individuals who are concerned about blood uric acid levels, individuals having higher uric acid levels, individuals who are concerned about uric acid levels, individuals who are concerned about purine products, individuals who want to prevent hyperuricemia, individuals who are concerned about hyperuricemia, individuals who want to prevent gout, individuals who are concerned about gout, and the like.

The composition of the present invention contains a specified amount of the cyclic dipeptide or a salt thereof, and includes the following three embodiments depending upon the applications of the composition. Here, the content of the cyclic dipeptide or a salt thereof in each of the embodiments can be measured in accordance with known methods, and, for example, the contents can be measured by subjecting to LC-MS/MS.

< Embodiment 1 >

Embodiment 1 is an extract composition containing a cyclic dipeptide or a salt thereof mentioned above. The extract composition refers to a composition which directly uses a product prepared by treating a cyclic dipeptide mentioned above or materials containing constituting amino acids of the cyclic dipeptide, or a composition containing the product subjected to dilution, concentration, or purification in accordance with a known method, and the extract composition can be produced into formulation, or used as raw materials for medicaments, quasi-drugs, or foodstuff (dietary supplements), and the like.

The contents of each of the cyclic dipeptides or salts thereof in Embodiment 1 are as follows.

(1) Cyclotryptophanyltyrosine or Salt Thereof

The content in the composition is preferably $0.08 \times 10$ ppm or more, more preferably $0.08 \times 10^2$ ppm or more, and even more preferably $0.16 \times 10^2$ ppm or more, and preferably $0.12 \times 10^5$ ppm or less, more preferably $0.12 \times 10^4$ ppm or less, and even more preferably $0.06 \times 10^4$ ppm or less.

(2) Cycloseryltyrosine or Salt Thereof

The content in the composition is preferably $0.80 \times 10$ ppm or more, more preferably $0.80 \times 10^2$ ppm or more, and even more preferably $1.60 \times 10^2$ ppm or more, and preferably $1.30 \times 10^5$ ppm or less, more preferably $1.30 \times 10^4$ ppm or less, and even more preferably $0.65 \times 10^4$ ppm or less.

(3) Cycloprolyltyrosine or Salt Thereof

The content in the composition is preferably $0.40 \times 10$ ppm or more, more preferably $0.40 \times 10^2$ ppm or more, and even more preferably $0.80 \times 10^2$ ppm or more, and preferably $0.70 \times 10^5$ ppm or less, more preferably $0.70 \times 10^4$ ppm or less, and even more preferably $0.35 \times 10^4$ ppm or less.

(4) Cyclotyrosylglycine or Salt Thereof

The content in the composition is preferably $0.30 \times 10$ ppm or more, more preferably $0.30 \times 10^2$ ppm or more, and even more preferably $0.60 \times 10^2$ ppm or more, and preferably $0.60 \times 10^5$ ppm or less, more preferably $0.60 \times 10^4$ ppm or less, and even more preferably $0.30 \times 10^4$ ppm or less.

(5) Cyclotyrosyltyrosine or Salt Thereof

The content in the composition is preferably $0.10 \times 10$ ppm or more, more preferably $0.10 \times 10^2$ ppm or more, and even more preferably $0.20 \times 10^2$ ppm or more, and preferably $0.30 \times 10^5$ ppm or less, more preferably $0.30 \times 10^4$ ppm or less, and even more preferably $0.15 \times 10^4$ ppm or less.

(6) Cyclophenylalanyltyrosine or Salt Thereof

The content in the composition is preferably $0.50 \times 10$ ppm or more, more preferably $0.50 \times 10^2$ ppm or more, and even more preferably $1.00 \times 10^2$ ppm or more, and preferably $0.90 \times 10^5$ ppm or less, more preferably $0.90 \times 10^4$ ppm or less, and even more preferably $0.45 \times 10^4$ ppm or less.

(7) Cycloleucyltyrosine or Salt Thereof

The content in the composition is preferably $0.70 \times 10$ ppm or more, $0.70 \times 10^2$ ppm or more, and even more preferably $1.40 \times 10^2$ ppm or more, and preferably $1.10 \times 10^5$ ppm or less, more preferably $1.10 \times 10^4$ ppm or less, and even more preferably $0.55 \times 10^4$ ppm or less.

(8) Cyclolysyltyrosine or Salt Thereof

The content in the composition is preferably $1.00 \times 10$ ppm or more, $1.00 \times 10^2$ ppm or more, and even more preferably $2.00 \times 10^2$ ppm or more, and preferably $1.50 \times 10^5$ ppm or less, more preferably $1.50 \times 10^4$ ppm or less, and even more preferably $0.75 \times 10^4$ ppm or less.

(9) Cyclohistidyltyrosine or Salt Thereof

The content in the composition is preferably $0.20 \times 10$ ppm or more, more preferably $0.20 \times 10^2$ ppm or more, and even more preferably $0.40 \times 10^2$ ppm or more, and preferably $0.40 \times 10^5$ ppm or less, more preferably $0.40 \times 10^4$ ppm or less, and even more preferably $0.20 \times 10^4$ ppm or less.

(10) Cycloalanyltyrosine or Salt Thereof

The content in the composition is preferably $0.40 \times 10$ ppm or more, $0.40 \times 10^2$ ppm or more, and even more preferably $0.80 \times 10^2$ ppm or more, and preferably $0.70 \times 10^5$ ppm or less, more preferably $0.70 \times 10^4$ ppm or less, and even more preferably $0.35 \times 10^4$ ppm or less.

(11) Cycloglutamyltyrosine or Salt Thereof

The content in the composition is preferably $0.10 \times 10$ ppm or more, more preferably $0.10 \times 10^2$ ppm or more, and even more preferably $0.20 \times 10^2$ ppm or more, and preferably $0.30 \times 10^5$ ppm or less, more preferably $0.30 \times 10^4$ ppm or less, and even more preferably $0.15 \times 10^4$ ppm or less.

(12) Cyclovalyltyrosine or Salt Thereof

The content in the composition is preferably $0.10 \times 10$ ppm or more, more preferably $0.10 \times 10^2$ ppm or more, and even more preferably $0.20 \times 10^2$ ppm or more, and preferably $0.30 \times 10^5$ ppm or less, more preferably $0.30 \times 10^4$ ppm or less, and even more preferably $0.15 \times 10^4$ ppm or less.

(13) Cycloisoleucyltyrosine or Salt Thereof

The content in the composition is preferably $0.80 \times 10$ ppm or more, more preferably $0.80 \times 10^2$ ppm or more, and even more preferably $1.60 \times 10^2$ ppm or more, and preferably $1.30 \times 10^5$ ppm or less, more preferably $1.30 \times 10^4$ ppm or less, and even more preferably $0.65 \times 10^4$ ppm or less.

(14) Cyclothreonyltyrosine or Salt Thereof

The content in the composition is preferably $0.50 \times 10$ ppm or more, more preferably $0.50 \times 10^2$ ppm or more, and even more preferably $1.00 \times 10^2$ ppm or more, and preferably $0.90 \times 10^5$ ppm or less, more preferably $0.90 \times 10^4$ ppm or less, and even more preferably $0.45 \times 10^4$ ppm or less.

(15) Cycloaspartyltyrosine or Salt Thereof

The content in the composition is preferably $0.10 \times 10$ ppm or more, more preferably $0.10 \times 10^2$ ppm or more, and even more preferably $0.20 \times 10^2$ ppm or more, and preferably $0.30 \times 10^5$ ppm or less, more preferably $0.30 \times 10^4$

ppm or less, and even more preferably $0.15 \times 10^4$ ppm or less.

(16) Cycloasparaginyltyrosine or Salt Thereof

The content in the composition is preferably $0.50 \times 10$ ppm or more, more preferably $0.50 \times 10^2$ ppm or more, and even more preferably $1.00 \times 10^2$ ppm or more, and preferably $0.90 \times 10^5$ ppm or less, more preferably $0.90 \times 10^4$ ppm or less, and even more preferably $0.45 \times 10^4$ ppm or less.

(17) Cycloglutaminyltyrosine or Salt Thereof

The content in the composition is preferably $0.30 \times 10$ ppm or more, more preferably $0.30 \times 10^2$ ppm or more, and even more preferably $0.60 \times 10^2$ ppm or more, and preferably $0.50 \times 10^5$ ppm or less, more preferably $0.50 \times 10^4$ ppm or less, and even more preferably $0.25 \times 10^4$ ppm or less.

(18) Cycloarginyltyrosine or Salt Thereof

The content in the composition is preferably $1.00 \times 10$ ppm or more, more preferably $1.00 \times 10^2$ ppm or more, and even more preferably $2.00 \times 10^2$ ppm or more, and preferably $1.60 \times 10^5$ ppm or less, more preferably $1.60 \times 10^4$ ppm or less, and even more preferably $0.80 \times 10^4$ ppm or less.

(19) Cyclomethionyltyrosine or Salt Thereof

The content in the composition is preferably $0.10 \times 10$ ppm or more, more preferably $0.10 \times 10^2$ ppm or more, and even more preferably $0.20 \times 10^2$ ppm or more, and preferably $0.20 \times 10^5$ ppm or less, more preferably $0.20 \times 10^4$ ppm or less, and even more preferably $0.10 \times 10^4$ ppm or less.

Among them, the cyclic dipeptide mentioned above or a salt thereof in Embodiment 1 may be any of the components so long as the content of at least one component falls within the range defined above. It is desired that preferably the content of one or more cyclic dipeptides selected from the group consisting of cycloseryltyrosine, cyclotyrosylglycine, cyclotyrosyltyrosine, cyclophenylalanyltyrosine, cycloleucyltyrosine, cyclovalyltyrosine, cycloisoleucyltyrosine, cycloaspartyltyrosine, cycloasparaginyltyrosine, cycloarginyltyrosine, and cyclomethionyltyrosine, or a salt thereof, more preferably the content of one or more cyclic dipeptides selected from the group consisting of cyclotyrosylglycine, cyclotyrosyltyrosine, cyclophenylalanyltyrosine, cycloleucyltyrosine, cyclovalyltyrosine, cycloisoleucyltyrosine, and cyclomethionyltyrosine, or a salt thereof, even more preferably the content of one or more cyclic dipeptides selected from the group consisting of cyclotyrosyltyrosine, cyclophenylalanyltyrosine, and cycloleucyltyrosine, or a salt thereof, and even more preferably the content of cyclotyrosyltyrosine or a salt thereof, falls within the range defined above, from the viewpoint of actions of inhibiting a xanthine oxidase.

In addition, it is desired that preferably the content of one or more cyclic dipeptides selected from the group consisting of cyclolysyltyrosine, cycloisoleucyltyrosine, cyclothreonyltyrosine, cycloaspartyltyrosine, cycloasparaginyltyrosine, cycloglutaminyltyrosine, cycloarginyltyrosine, and cyclomethionyltyrosine, or a salt thereof, more preferably the content of one or more cyclic dipeptides selected from the group consisting of cyclolysyltyrosine, cycloisoleucyltyrosine, cyclothreonyltyrosine, cycloaspartyltyrosine, cycloglutaminyltyrosine, and cycloarginyltyrosine, or a salt thereof, and even more preferably the content of one or more cyclic dipeptides selected from the group consisting of cyclolysyltyrosine, cycloisoleucyltyrosine, cyclothreonyltyrosine, and cycloarginyltyrosine, or a salt thereof, falls within the range defined above, from the viewpoint of solubility in water. In addition, the component to be combined with the above-mentioned component may include any of the components, and it is desired that preferably the content of one or more cyclic dipeptides selected from the group consisting of cycloseryltyrosine, cyclotyrosylglycine, cyclotyrosyltyrosine, cyclophenylalanyltyrosine, cycloleucyltyrosine, and cyclovalyltyrosine, or a salt thereof, more preferably the content of one or more cyclic dipeptides selected from the group consisting of cyclotyrosylglycine, cyclotyrosyltyrosine, cyclophenylalanyltyrosine, cycloleucyltyrosine, and cyclovalyltyrosine, or a salt thereof, even more preferably the content of one or more cyclic dipeptides selected from the group consisting of cyclotyrosyltyrosine, cyclophenylalanyltyrosine, and cycloleucyltyrosine, or a salt thereof, and even more preferably the content of cyclotyrosyltyrosine or a salt thereof, falls within the range defined above, from the viewpoint of inhibiting actions of a xanthine oxidase.

Also, it is desired that the number of the components satisfying the content of each component listed above is preferably one component or more, more preferably 3 components or more, even more preferably 4 components or more, even more preferably 6 components or more, even more preferably 7 components or more, even more preferably 8 components or more, and even more preferably 11 components or more. Concretely, for example, preferably the contents of cyclotyrosyltyrosine, cyclophenylalanyltyrosine, and cycloleucyltyrosine fall within the range defined above, more preferably the contents of cyclotyrosylglycine, cyclotyrosyltyrosine, cyclophenylalanyltyrosine, cycloleucyltyrosine, cyclovalyltyrosine, cycloisoleucyltyrosine, and cyclomethionyltyrosine fall within the range defined above, and even more preferably the contents of cycloseryltyrosine, cyclotyrosylglycine, cyclotyrosyltyrosine, cyclophenylalanyltyrosine, cycloleucyltyrosine, cyclovalyltyrosine, cycloisoleucyltyrosine, cycloaspartyltyrosine, cycloasparaginyltyrosine, cycloarginyltyrosine, and cyclomethionyltyrosine fall within the range defined above, from the viewpoint of inhibiting actions of a xanthine oxidase. In addition, preferably the contents of cyclolysyltyrosine, cycloisoleucyltyrosine, cyclothreonyltyrosine, and cycloarginyltyrosine fall within the range defined above, more preferably the contents of cyclolysyltyrosine, cycloisoleucyltyrosine, cyclothreonyltyrosine, cycloaspartyltyrosine, cycloglutaminyltyrosine, and cycloarginyltyrosine fall within the range defined above, and even more preferably the contents of cyclolysyltyrosine, cycloisoleucyltyrosine, cyclothreonyltyrosine, cycloaspartyltyrosine, cycloasparaginyltyrosine, cycloglutaminyltyrosine, cycloarginyltyrosine, and cyclomethionyltyrosine fall within the range defined above, from the viewpoint of solubility in water.

The contents of the above-mentioned cyclic dipeptides or a salt thereof in Embodiment 1 are as mentioned above for each component, and a total content of the cyclic dipeptide or a salt thereof is preferably $8.0 \times 10$ ppm or more, more preferably $8.0 \times 10^2$ ppm or more, and even more preferably $1.6 \times 10^3$ ppm or more, and preferably $1.0 \times 10^6$ ppm or less, more preferably $1.4 \times 10^5$ ppm or less, and even more preferably $0.7 \times 10^5$ ppm or less.

In addition, a total content of cyclotyrosyltyrosine, cyclophenylalanyltyrosine, cycloleucyltyrosine, and salts thereof, each having a high action of inhibiting a xanthine oxidase, in Embodiment 1 is preferably $1.3 \times 10$ ppm or more, more preferably $1.3 \times 10^2$ ppm or more, and even more preferably $2.6 \times 10^2$ ppm or more, and preferably $2.3 \times 10^5$ ppm or less, more preferably $2.3 \times 10^4$ ppm or less, and even more preferably $1.15 \times 10^4$ ppm or less.

Among the cyclic dipeptides in Embodiment 1, a proportion of the total amount occupied by cyclotyrosyltyrosine, cyclophenylalanyltyrosine, and cycloleucyltyrosine, and salts thereof, each having a high action of inhibiting a xanthine oxidase is preferably 5% by weight or more, more preferably 10% by weight or more, and even more preferably 15% by weight or more, from the viewpoint of the effects of inhibiting a xanthine oxidase. In addition, although the upper limit is not particularly limited, the proportion is preferably 90% by weight or less, and more preferably 60% by weight or less, from the viewpoint of solubilization property.

In addition, a weight ratio of cyclophenylalanyltyrosine to cyclotyrosyltyrosine [Cyclo(Tyr-Phe)/Cyclo(Tyr-Tyr)] is preferably from 95/5 to 35/65, more preferably from 90/10 to 40/60, and even more preferably from 90/10 to 50/50, from the viewpoint of the effects of inhibiting a xanthine oxidase.

Also, a weight ratio of cyclophenylalanyltyrosine to cyclotyrosylglycine [Cyclo(Phe-Tyr)/Cyclo(Tyr-Gly)] is preferably from 90/10 to 30/70, more preferably from 90/10 to 40/60, and even more preferably from 90/10 to 50/50, from the viewpoint of the effects of inhibiting a xanthine oxidase.

In addition, a total content of cyclolysyltyrosine, cycloarginyltyrosine, and salts thereof, each having a high solubility in water, in Embodiment 1 is preferably $2.00 \times 10$ ppm or more, more preferably $2.00 \times 10^2$ ppm or more, and even more preferably $4.00 \times 10^2$ ppm or more, and preferably $3.10 \times 10^5$ ppm or less, more preferably $3.10 \times 10^4$ ppm or less, and even more preferably $1.55 \times 10^4$ ppm or less.

Among the cyclic dipeptides in Embodiment 1, a proportion of the total amount occupied by cyclolysyltyrosine, cycloarginyltyrosine, and salts thereof, each having a high solubility in water, is preferably 5% by weight or more, more preferably 10% by weight or more, and even more preferably 15% by weight or more, from the viewpoint of preparing a composition. In addition, although the upper limit is not particularly limited, the proportion of a total amount is preferably 90% by weight or less, and more preferably 60% by weight or less, from the viewpoint of solubilization property.

The composition of Embodiment 1 can be produced into a formulation in the form of a solid such as a tablet, a granule, a powder, a fine powder, or a capsule, or a liquid such as a common liquid agent, a suspension agent or an emulsion agent in accordance with a known method by adding as desired a solvent, a dispersant, an emulsifier, a buffer, a stabilizer, an excipient, a binder, a disintegrant, a lubricant or the like to raw materials containing a cyclic dipeptide or a salt thereof mentioned above. These compositions can be directly taken together with water or the like. In addition, these compositions can be used as raw materials for, for example, medicaments, quasi-drugs, or foodstuff (dietary supplements), and the like after preparing the compositions into a form that can be easily blended, for example, a powder form or a granular form.

Concrete examples of the composition of Embodiment 1 include a solid such as a tablet, a granule, a powder, a fine powder, or a capsule, or a liquid such as a common liquid agent, a suspension agent or an emulsion agent.

< Embodiment 2 >

Embodiment 2 is a beverage composition containing a cyclic dipeptide or a salt thereof mentioned above. The beverage composition refers to a composition usable in drinking applications, and the contents of each of the cyclic dipeptides or salts thereof in Embodiment 2 are as follows.

(1) Cyclotryptophanyltyrosine or Salt Thereof

The content in the composition is preferably $0.4 \times 10^{-4}$ mg/100 mL or more, more preferably $0.4 \times 10^{-3}$ mg/100 mL or more, and even more preferably $0.8 \times 10^{-3}$ mg/100 mL or more, and preferably $0.7 \times 10$ mg/100 mL or less, more preferably 0.7 mg/100 mL or less, and even more preferably $3.5 \times 10^{-1}$ mg/100 mL or less.

(2) Cycloseryltyrosine or Salt Thereof

The content in the composition is preferably $4.0 \times 10^{-4}$ mg/100 mL or more, more preferably $4.0 \times 10^{-3}$ mg/100 mL or more, and even more preferably $8.0 \times 10^{-3}$ mg/100 mL or more, and preferably $8.0 \times 10$ mg/100 mL or less, more preferably 8.0 mg/100 mL or less, and even more preferably 4.0 mg/100 mL or less.

(3) Cycloprolyltyrosine or Salt Thereof

The content in the composition is preferably $2.0 \times 10^{-4}$ mg/100 mL or more, more preferably $2.0 \times 10^{-3}$ mg/100 mL or more, and even more preferably $4.0 \times 10^{-3}$ mg/100 mL or more, and preferably $4.0 \times 10$ mg/100 mL or less, more preferably 4.0 mg/100 mL or less, and even more preferably 2.0 mg/100 mL or less.

(4) Cyclotyrosylglycine or Salt Thereof

The content in the composition is preferably $2.0 \times 10^{-4}$ mg/100 mL or more, more preferably $2.0 \times 10^{-3}$ mg/100 mL or more, and even more preferably $4.0 \times 10^{-3}$ mg/100 mL or more, and preferably $4.0 \times 10$ mg/100 mL or less, more preferably 4.0 mg/100 mL or less, and even more preferably 2.0 mg/100 mL or less.

(5) Cyclotyrosyltyrosine or Salt Thereof

The content in the composition is preferably $0.8 \times 10^{-4}$ mg/100 mL or more, more preferably $0.8 \times 10^{-3}$ mg/100 mL or more, and even more preferably $1.6 \times 10^{-3}$ mg/100 mL or more, and preferably $2.0 \times 10$ mg/100 mL or less, more preferably 2.0 mg/100 mL or less, and even more preferably 1.0 mg/100 mL or less.

(6) Cyclophenylalanyltyrosine or Salt Thereof

The content in the composition is preferably $3.0 \times 10^{-4}$ mg/100 mL or more, more preferably $3.0 \times 10^{-3}$ mg/100 mL or more, and even more preferably $6.0 \times 10^{-3}$ mg/100 mL or more, and preferably $5.0 \times 10$ mg/100 mL or less, more preferably 5.0 mg/100 mL or less, and even more preferably 2.5 mg/100 mL or less.

(7) Cycloleucyltyrosine or Salt Thereof

The content in the composition is preferably $4.0 \times 10^{-4}$ mg/100 mL or more, more preferably $4.0 \times 10^{-3}$ mg/100 mL or more, and even more preferably $8.0 \times 10^{-3}$ mg/100 mL or more, and preferably $7.0 \times 10$ mg/100 mL or less, more preferably 7.0 mg/100 mL or less, and even more preferably 3.5 mg/100 mL or less.

(8) Cyclolysyltyrosine or Salt Thereof

The content in the composition is preferably $5.0 \times 10^{-4}$ mg/100 mL or more, more preferably $5.0 \times 10^{-3}$ mg/100 mL or more, and even more preferably $1.0 \times 10^{-2}$ mg/100 mL or more, and preferably $9.0 \times 10$ mg/100 mL or less, more preferably 9.0 mg/100 mL or less, and even more preferably 4.5 mg/100 mL or less.

(9) Cyclohistidyltyrosine or Salt Thereof

The content in the composition is preferably $1.0 \times 10^{-4}$ mg/100 mL or more, more preferably $1.0 \times 10^{-3}$ mg/100 mL or more, and even more preferably $2.0 \times 10^{-3}$ mg/100 mL or more, and preferably $3.0 \times 10$ mg/100 mL or less, more preferably 3.0 mg/100 mL or less, and even more preferably 1.5 mg/100 mL or less.

(10) Cycloalanyltyrosine or Salt Thereof

The content in the composition is preferably $2.0 \times 10^{-4}$ mg/100 mL or more, more preferably $2.0 \times 10^{-3}$ mg/100 mL or more, and even more preferably $4.0 \times 10^{-3}$ mg/100 mL or more, and preferably $4.0 \times 10$ mg/100 mL or less, more preferably 4.0 mg/100 mL or less, and even more preferably 2.0 mg/100 mL or less.

(11) Cycloglutamyltyrosine or Salt Thereof

The content in the composition is preferably $1.0 \times 10^{-4}$ mg/100 mL or more, more preferably $1.0 \times 10^{-3}$ mg/100 mL or more, and even more preferably $2.0 \times 10^{-3}$ mg/100 mL or more, and preferably $2.0 \times 10$ mg/100 mL or less, more preferably 2.0 mg/100 mL or less, and even more preferably 1.0 mg/100 mL or less.

(12) Cyclovalyltyrosine or Salt Thereof

The content in the composition is preferably $1.0 \times 10^{-4}$ mg/100 mL or more, more preferably $1.0 \times 10^{-3}$ mg/100 mL or more, and even more preferably $2.0 \times 10^{-3}$ mg/100 mL or more, and preferably $2.0 \times 10$ mg/100 mL or less, more preferably 2.0 mg/100 mL or less, and even more preferably 1.0 mg/100 mL or less.

(13) Cycloisoleucyltyrosine or Salt Thereof

The content in the composition is preferably $4.0 \times 10^{-4}$ mg/100 mL or more, more preferably $4.0 \times 10^{-3}$ mg/100 mL or more, and even more preferably $8.0 \times 10^{-3}$ mg/100 mL or more, and preferably $7.0 \times 10$ mg/100 mL or less, more preferably 7.0 mg/100 mL or less, and even more preferably 3.5 mg/100 mL or less.

(14) Cyclothreonyltyrosine or Salt Thereof

The content in the composition is preferably $3.0 \times 10^{-4}$ mg/100 mL or more, more preferably $3.0 \times 10^{-3}$ mg/100 mL or more, and even more preferably $6.0 \times 10^{-3}$ mg/100 mL or more, and preferably $5.0 \times 10$ mg/100 mL or less, more preferably 5.0 mg/100 mL or less, and even more preferably 2.5 mg/100 mL or less.

(15) Cycloaspartyltyrosine or Salt Thereof

The content in the composition is preferably $1.0 \times 10^{-4}$ mg/100 mL or more, more preferably $1.0 \times 10^{-3}$ mg/100 mL or more, and even more preferably $2.0 \times 10^{-3}$ mg/100 mL or more, and preferably $2.0 \times 10$ mg/100 mL or less, more preferably 2.0 mg/100 mL or less, and even more preferably 1.0 mg/100 mL or less.

(16) Cycloasparaginyltyrosine or Salt Thereof

The content in the composition is preferably $3.0 \times 10^{-4}$ mg/100 mL or more, more preferably $3.0 \times 10^{-3}$ mg/100 mL or more, and even more preferably $6.0 \times 10^{-3}$ mg/100 mL or more, and preferably $5.0 \times 10$ mg/100 mL or less, more preferably 5.0 mg/100 mL or less, and even more preferably 2.5 mg/100 mL or less.

(17) Cycloglutaminyltyrosine or Salt Thereof

The content in the composition is preferably $1.0 \times 10^{-4}$ mg/100 mL or more, more preferably $1.0 \times 10^{-3}$ mg/100 mL or more, and even more preferably $2.0 \times 10^{-3}$ mg/100 mL or more, and preferably $3.0 \times 10$ mg/100 mL or less, more preferably 3.0 mg/100 mL or less, and even more preferably 1.5 mg/100 mL or less.

(18) Cycloarginyltyrosine or Salt Thereof

The content in the composition is preferably $6.0 \times 10^{-4}$ mg/100 mL or more, more preferably $6.0 \times 10^{-3}$ mg/100 mL or more, and even more preferably $1.2 \times 10^{-2}$ mg/100 mL or more, and preferably $1.0 \times 10^{2}$ mg/100 mL or less, more preferably $1.0 \times 10$ mg/100 mL or less, and even more preferably 5.0 mg/100 mL or less.

(19) Cyclomethionyltyrosine or Salt Thereof

The content in the composition is preferably $0.7 \times 10^{-4}$ mg/100 mL or more, more preferably $0.7 \times 10^{-3}$ mg/100 mL or more, and even more preferably $1.4 \times 10^{-3}$ mg/100 mL or more, and preferably $2.0 \times 10$ mg/100 mL or less, more preferably 2.0 mg/100 mL or less, and even more preferably 1.0 mg/100 mL or less.

Among them, the cyclic dipeptide mentioned above or a salt thereof in Embodiment 2 may be any of the components so long as the content of at least one component falls within the range defined above. It is desired that preferably the content of one or more cyclic dipeptides selected from the group consisting of cycloseryltyrosine, cyclotyrosylglycine, cyclotyrosyltyrosine, cyclophenylalanyltyrosine, cycloleucyltyrosine, cyclovalyltyrosine, cycloisoleucyltyrosine, cycloaspartyltyrosine, cycloasparaginyltyrosine, cycloarginyltyrosine, and cyclomethionyltyrosine, or a salt thereof, more preferably the content of one or more cyclic dipeptides selected from the group consisting of cyclotyrosylglycine, cyclotyrosyltyrosine, cyclophenylalanyltyrosine, cycloleucyltyrosine, cyclovalyltyrosine, cycloisoleucyltyrosine, and cyclomethionyltyrosine, or a salt thereof, even more preferably the content of one or more cyclic dipeptides selected from the group consisting of cyclotyrosyltyrosine, cyclophenylalanyltyrosine, and cycloleucyltyrosine, or a salt thereof, and even more preferably the content of cyclotyrosyltyrosine or a salt thereof, falls within the range defined above, from the viewpoint of actions of inhibiting a xanthine oxidase.

In addition, it is desired that preferably the content of one or more cyclic dipeptides selected from the group consisting of cyclolysyltyrosine, cycloisoleucyltyrosine, cyclothreonyltyrosine, cycloaspartyltyrosine, cycloasparaginyltyrosine, cycloglutaminyltyrosine, cycloarginyltyrosine, and cyclomethionyltyrosine, or a salt thereof, more preferably the content of one or more cyclic dipeptides selected from the group consisting of cyclolysyltyrosine, cycloisoleucyltyrosine, cyclothreonyltyrosine, cycloaspartyltyrosine, cycloglutaminyltyrosine, and cycloarginyltyrosine, or a salt thereof, and even more preferably the content of one or more cyclic dipeptides selected from the group consisting of cyclolysyltyrosine, cycloisoleucyltyrosine, cyclothreonyltyrosine, and cycloarginyltyrosine, or a salt thereof, falls within the range defined above, from the viewpoint of solubility in water. In addition, the component to be combined with the above-mentioned component may include any of the components, and it is desired that preferably the content of one or more cyclic dipeptides selected from the group consisting of cycloseryltyrosine, cyclotyrosylglycine, cyclotyrosyltyrosine, cyclophenylalanyltyrosine, cycloleucyltyrosine, and cyclovalyltyrosine, or a salt thereof, more preferably the content of one or more cyclic dipeptides selected from the group consisting of cyclotyrosylglycine, cyclotyrosyltyrosine, cyclophenylalanyltyrosine, cycloleucyltyrosine, and cyclovalyltyrosine, or a salt thereof, even more preferably the content of one or more cyclic dipeptides selected from the group consisting of cyclotyrosyltyrosine, cyclophenylalanyltyrosine, and cycloleucyltyrosine, or a salt thereof, and even more preferably the content of cyclotyrosyltyrosine or a salt thereof, falls within the range defined above, from the viewpoint of inhibiting actions of a xanthine oxidase.

Also, it is desired that the number of the components satisfying the content of each component listed above is preferably one component or more, more preferably 3 components or more, even more preferably 4 components or more, even more preferably 6 components or more, even more preferably 7 components or more, even more preferably 8 components or more, and even more preferably 11 components or more. Concretely, for example, preferably the contents of cyclotyrosyltyrosine, cyclophenylalanyltyrosine, and cycloleucyltyrosine fall within the range defined above, more preferably the contents of cyclotyrosylglycine, cyclotyrosyltyrosine, cyclophenylalanyltyrosine, cycloleucyltyrosine, cyclovalyltyrosine, cycloisoleucyltyrosine, and cyclomethionyltyrosine fall within the range defined above, and even more preferably the contents of cycloseryltyrosine, cyclotyrosylglycine, cyclotyrosyltyrosine, cyclophenylalanyltyrosine, cycloleucyltyrosine, cyclovalyltyrosine, cycloisoleucyltyrosine, cycloaspartyltyrosine, cycloasparaginyltyrosine, cycloarginyltyrosine, and cyclomethionyltyrosine fall within the range defined above, from the viewpoint of inhibiting actions of a xanthine oxidase. In addition, preferably the contents of cyclolysyltyrosine, cycloisoleucyltyrosine, cyclothreonyltyrosine, and cycloarginyltyrosine fall within the range defined above, more preferably the contents of cyclolysyltyrosine, cycloisoleucyltyrosine, cyclothreonyltyrosine, cycloaspartyltyrosine, cycloglutaminyltyrosine, and cycloarginyltyrosine fall within the range defined above, and even more preferably the contents of cyclolysyltyrosine, cycloisoleucyltyrosine, cyclothreonyltyrosine, cycloaspartyltyrosine, cycloasparaginyltyrosine, cycloglutaminyltyrosine, cycloarginyltyrosine, and cyclomethionyltyrosine fall within the range defined above, from the viewpoint of solubility in water.

The contents of the above-mentioned cyclic dipeptide or a salt thereof in Embodiment 2 are as mentioned above for each component, and a total content of the cyclic dipeptide or a salt thereof is preferably $5.0 \times 10^{-3}$ mg/100 mL or more, more preferably $5.0 \times 10^{-2}$ mg/100 mL or more, and even more preferably $1.0 \times 10^{-1}$ mg/100 mL or more, and preferably $8.0 \times 10^2$ mg/100 mL or less, more preferably $8.0 \times 10$ mg/100 mL or less, and even more preferably $4.0 \times 10$ mg/100 mL or less.

In addition, a total content of cyclotyrosyltyrosine, cyclophenylalanyltyrosine, cycloleucyltyrosine, and salts thereof, each having a high action of inhibiting a xanthine oxidase, in Embodiment 2 is preferably $7.8 \times 10^{-4}$ mg/100 mL or more, more preferably $7.8 \times 10^{-3}$ mg/100 mL or more, and even more preferably $1.56 \times 10^{-2}$ mg/100 mL or more, and preferably $1.4 \times 10$ mg/100 mL or less, more preferably 1.4 mg/100 mL or less, and even more preferably 0.7 mg/100 mL or less.

Among the cyclic dipeptides in Embodiment 2, a proportion of the total amount occupied by cyclotyrosyltyrosine, cyclophenylalanyltyrosine, cycloleucyltyrosine, and salts thereof, each having a high action of inhibiting a xanthine oxidase, is preferably 5% by weight or more, more preferably 10% by weight or more, and even more preferably 15% by weight or more, from the viewpoint of the effects of inhibiting a xanthine oxidase. In addition, although the upper limit is not particularly limited, the proportion is preferably 90% by weight or less, and more preferably 60% by weight or less, from the viewpoint of solubilization property.

In addition, a weight ratio of cyclophenylalanyltyrosine to cyclotyrosyltyrosine [Cyclo(Tyr-Phe)/Cyclo(Tyr-Tyr)] is preferably from 95/5 to 35/65, more preferably from 90/10 to 40/60, and even more preferably from 90/10 to 50/50, from the viewpoint of the effects of inhibiting a xanthine oxidase.

Also, a weight ratio of cyclophenylalanyltyrosine to cyclotyrosylglycine [Cyclo(Phe-Tyr)/Cyclo(Tyr-Gly)] is preferably from 90/10 to 30/70, more preferably from 90/10 to 40/60, and even more preferably from 90/10 to 50/50, from the viewpoint of the effects of inhibiting a xanthine oxidase.

In addition, a total content of cyclolysyltyrosine, cycloarginyltyrosine, and salts thereof, each having a high solubility in water, in Embodiment 2 is preferably $11.0 \times 10^{-4}$ mg/100 mL or more, more preferably $11.0 \times 10^{-3}$ mg/100 mL or more, and even more preferably $2.2 \times 10^{-2}$ mg/100 mL or more, and preferably $19.0 \times 10$ mg/100 mL or less, more preferably 19.0 mg/100 mL or less, and even more preferably 9.5 mg/100 mL or less.

Among the cyclic dipeptides in Embodiment 2, a proportion of a total amount occupied by cyclolysyltyrosine, cycloarginyltyrosine, and salts thereof, each having a high solubility in water, is preferably 5% by weight or more, more preferably 10% by weight or more, and even more preferably 15% by weight or more, from the viewpoint of preparing a composition. In addition, although the upper limit is not particularly limited, the proportion of a total amount is preferably 90% by weight or less, and more preferably 60% by weight or less, from the viewpoint of solubilization property.

The composition of Embodiment 2 may be prepared by, for example, when preparing a known beverage composition, mixing a given amount of a cyclic dipeptide or a salt thereof mentioned above to the raw materials of the beverage composition, to prepare a beverage composition in accordance with a known method for producing a beverage composition. Alternatively, the composition can be prepared by adding the above cyclic dipeptide or a salt thereof to an already made known beverage composition so as to be contained a given amount mentioned above. Here, a known beverage composition may be those originally containing the cyclic dipeptide or a salt thereof, or if the cyclic dipeptide of the present invention is contained in a given amount, the cyclic dipeptide may be properly blended to prepare the beverage composition.

Specific examples of the composition of Embodiment 2 include non-alcoholic beverages such as oolong tea beverages, tea beverages, green tea beverages, fruit juice beverages, vegetable juices, sports drinks, isotonic beverages, enhanced water, mineral water, near water, coffee beverages, nutritional drink agents, cosmetic drink agents, and nonalcoholic beer-taste beverages; alcoholic beverages such as beer, wine, sake, plum wine, *happoshu* (low-malt beer-like beverage), whisky, brandy, *shochu* (distilled spirits), rum, gin, and liqueurs. In addition, the composition of Embodiment 2 may be blended with an additive such as an antioxidant, a flavor, an organic acid, a salt of an organic acid, an inorganic acid, a salt of an inorganic acid, an inorganic salt, a pigment, an emulsifying agent, a preservative, a seasoning, a sweetener, an acidulant, a gum, an oil, a vitamin, an amino acid, a fruit extract, a vegetable extract, a pH adjusting agent, or a quality stabilizer, alone or in a combination thereof.

< Embodiment 3 >

Embodiment 3 is a food composition containing a cyclic dipeptide or a salt thereof mentioned above. The food composition refers to a composition usable in the ingestions mainly by meals and snacks, and the contents of each of the cyclic dipeptides or salts thereof in Embodiment 3 are as follows.

(1) Cyclotryptophanyltyrosine or Salt Thereof

The content in the composition is preferably 0.0008% by weight or more, more preferably 0.008% by weight or more, and even more preferably 0.016% by weight or more, and preferably 1.2% by weight or less, more preferably 0.12% by weight or less, and even more preferably 0.06% by weight or less.

(2) Cycloseryltyrosine or Salt Thereof

The content in the composition is preferably 0.008% by weight or more, more preferably 0.08% by weight or more, and even more preferably 0.16% by weight or more, and preferably 13% by weight or less, more preferably 1.3% by weight or less, and even more preferably 0.65% by weight or less.

(3) Cycloprolyltyrosine or Salt Thereof

The content in the composition is preferably 0.004% by weight or more, more preferably 0.04% by weight or more, and even more preferably 0.08% by weight or more, and preferably 7% by weight or less, more preferably 0.7% by weight or less, and even more preferably 0.35% by weight or less.

(4) Cyclotyrosylglycine or Salt Thereof

The content in the composition is preferably 0.003% by weight or more, more preferably 0.03% by weight or more, and even more preferably 0.06% by weight or more, and preferably 6% by weight or less, more preferably 0.6% by weight or less, and even more preferably 0.3% by weight or less.

(5) Cyclotyrosyltyrosine or Salt Thereof

The content in the composition is preferably 0.001% by weight or more, more preferably 0.01% by weight or more, and even more preferably 0.02% by weight or more, and preferably 3% by weight or less, more preferably 0.3% by weight or less, and even more preferably 0.15% by weight or less.

(6) Cyclophenylalanyltyrosine or Salt Thereof

The content in the composition is preferably 0.005% by weight or more, more preferably 0.05% by weight or more, and even more preferably 0.10% by weight or more, and preferably 9% by weight or less, more preferably 0.9% by weight or less, and even more preferably 0.45% by weight or less.

(7) Cycloleucyltyrosine or Salt Thereof

The content in the composition is preferably 0.007% by weight or more, more preferably 0.07% by weight or more, and even more preferably 0.14% by weight or more, and preferably 11% by weight or less, more preferably 1.1% by weight or less, and even more preferably 0.55% by weight or less.

(8) Cyclolysyltyrosine or Salt Thereof

The content in the composition is preferably 0.01% by weight or more, more preferably 0.1 % by weight or more, and even more preferably 0.2% by weight or more, and preferably 15% by weight or less, more preferably 1.5% by weight or less, and even more preferably 0.75% by weight or less.

(9) Cyclohistidyltyrosine or Salt Thereof

The content in the composition is preferably 0.002% by weight or more, more preferably 0.02% by weight or more, and even more preferably 0.04% by weight or more, and preferably 4% by weight or less, more preferably 0.4% by weight or less, and even more preferably 0.2% by weight or less.

(10) Cycloalanyltyrosine or Salt Thereof

The content in the composition is preferably 0.004% by weight or more, more preferably 0.04% by weight or more, and even more preferably 0.08% by weight or more, and preferably 7% by weight or less, more preferably 0.7% by weight or less, and even more preferably 0.35% by weight or less.

(11) Cycloglutamyltyrosine or Salt Thereof

The content in the composition is preferably 0.001% by weight or more, more preferably 0.01% by weight or more, and even more preferably 0.02% by weight or more, and preferably 3% by weight or less, more preferably 0.3% by weight or less, and even more preferably 0.15% by weight or less.

(12) Cyclovalyltyrosine or Salt Thereof

The content in the composition is preferably 0.001% by weight or more, more preferably 0.01% by weight or more, and even more preferably 0.02% by weight or more, and preferably 3% by weight or less, more preferably 0.3% by weight or less, and even more preferably 0.15% by weight or less.

(13) Cycloisoleucyltyrosine or Salt Thereof

The content in the composition is preferably 0.008% by weight or more, more preferably 0.08% by weight or more, and even more preferably 0.16% by weight or more, and preferably 13% by weight or less, more preferably 1.3% by weight or less, and even more preferably 0.65% by weight or less.

(14) Cyclothreonyltyrosine or Salt Thereof

The content in the composition is preferably 0.005% by weight or more, more preferably 0.05% by weight or more, and even more preferably 0.10% by weight or more, and preferably 9% by weight or less, more preferably 0.9% by weight or less, and even more preferably 0.45% by weight or less.

(15) Cycloaspartyltyrosine or Salt Thereof

The content in the composition is preferably 0.001% by weight or more, more preferably 0.01% by weight or more, and even more preferably 0.02% by weight or more, and preferably 3% by weight or less, more preferably 0.3% by weight or less, and even more preferably 0.15% by weight or less.

(16) Cycloasparaginyltyrosine or Salt Thereof

The content in the composition is preferably 0.005% by weight or more, more preferably 0.05% by weight or more, and even more preferably 0.10% by weight or more, and preferably 9% by weight or less, more preferably 0.9% by weight or less, and even more preferably 0.45% by weight or less.

(17) Cycloglutaminyltyrosine or Salt Thereof

The content in the composition is preferably 0.003% by weight or more, more preferably 0.03% by weight or more, and even more preferably 0.06% by weight or more, and preferably 5% by weight or less, more preferably 0.5% by weight or less, and even more preferably 0.25% by weight or less.

(18) Cycloarginyltyrosine or Salt Thereof

The content in the composition is preferably 0.01% by weight or more, more preferably 0.1% by weight or more, and even more preferably 0.2% by weight or more, and preferably 16% by weight or less, more preferably 1.6% by weight or less, and even more preferably 0.8% by weight or less.

(19) Cyclomethionyltyrosine or Salt Thereof

The content in the composition is preferably 0.001% by weight or more, more preferably 0.01 % by weight or more, and even more preferably 0.02% by weight or more, and preferably 2% by weight or less, more preferably 0.2% by weight or less, and even more preferably 0.1% by weight or less.

Among them, the cyclic dipeptide mentioned above or a salt thereof in Embodiment 3 may be any of the components so long as the content of at least one component falls within the range defined above. It is desired that preferably the content of one or more cyclic dipeptides selected from the group consisting of cycloseryltyrosine, cyclotyrosylglycine, cyclotyrosyltyrosine, cyclophenylalanyltyrosine, cycloleucyltyrosine, cyclovalyltyrosine, cycloisoleucyltyrosine, cycloaspartyltyrosine, cycloasparaginyltyrosine, cycloarginyltyrosine, and cyclomethionyltyrosine, or a salt thereof, more preferably the content of one or more cyclic dipeptides selected from the group consisting of cyclotyrosylglycine, cyclotyrosyltyrosine, cyclophenylalanyltyrosine, cycloleucyltyrosine, cyclovalyltyrosine, cycloisoleucyltyrosine, and cyclomethionyltyrosine, or a salt thereof, even more preferably the content of one or more cyclic dipeptides selected from the group consisting of cyclotyrosyltyrosine, cyclophenylalanyltyrosine, and cycloleucyltyrosine, or a salt thereof, and even more preferably the content of cyclotyrosyltyrosine or a salt thereof, falls within the range defined above, from the viewpoint of actions of inhibiting a xanthine oxidase.

In addition, it is desired that preferably the content of one or more cyclic dipeptides selected from the group consisting of cyclolysyltyrosine, cycloisoleucyltyrosine, cyclothreonyltyrosine, cycloaspartyltyrosine, cycloasparaginyltyrosine, cycloglutaminyltyrosine, cycloarginyltyrosine, and cyclomethionyltyrosine, or a salt thereof, more preferably the content of one or more cyclic dipeptides selected from the group consisting of cyclolysyltyrosine, cycloisoleucyltyrosine, cyclothreonyltyrosine, cycloaspartyltyrosine, cycloglutaminyltyrosine, and cycloarginyltyrosine, or a salt thereof, and even more preferably the content of one or more cyclic dipeptides selected from the group consisting of cyclolysyltyrosine, cycloisoleucyltyrosine, cyclothreonyltyrosine, and cycloarginyltyrosine, or a salt thereof, falls within the range defined above, from the viewpoint of solubility in water. In addition, the component to be combined with the abovementioned component may include any of the components, and it is desired that preferably the content of one or more cyclic dipeptides selected from the group consisting of cycloseryltyrosine, cyclotyrosylglycine, cyclotyrosyltyrosine, cyclophenylalanyltyrosine, cycloleucyltyrosine, and cyclovalyltyrosine, or a salt thereof, more preferably the content of one or more cyclic dipeptides selected from the group consisting of cyclotyrosylglycine, cyclotyrosyltyrosine, cyclophenylalanyltyrosine, cycloleucyltyrosine, and cyclovalyltyrosine, or a salt thereof, even more preferably the content of one or more cyclic dipeptides selected from the group consisting of cyclotyrosyltyrosine, cyclophenylalanyltyrosine, and cycloleucyltyrosine, or a salt thereof, and even more preferably the content of cyclotyrosyltyrosine or a salt thereof, falls within the range defined above, from the viewpoint of inhibiting actions of a xanthine oxidase.

Also, it is desired that the number of the components satisfying the content of each component listed above is preferably one component or more, more preferably 3 components or more, even more preferably 4 components or more, even more preferably 6 components or more, even more preferably 7 components or more, even more preferably 8 components or more, and even more preferably 11 components or more. Concretely, for example, preferably the contents of cyclotyrosyltyrosine, cyclophenylalanyltyrosine, and cycloleucyltyrosine fall within the range defined above, more preferably the contents of cyclotyrosylglycine, cyclotyrosyltyrosine, cyclophenylalanyltyrosine, cycloleucyltyrosine, cyclovalyltyrosine, cycloisoleucyltyrosine, and cyclomethionyltyrosine fall within the range defined above, and even more preferably the contents of cycloseryltyrosine, cyclotyrosylglycine, cyclotyrosyltyrosine, cyclophenylalanyltyrosine, cycloleucyltyrosine, cyclovalyltyrosine, cycloisoleucyltyrosine, cycloaspartyltyrosine, cycloasparaginyltyrosine, cycloarginyltyrosine, and cyclomethionyltyrosine fall within the range defined above, from the viewpoint of actions of inhibiting a xanthine oxidase. In addition, preferably the contents of cyclolysyltyrosine, cycloisoleucyltyrosine, cyclothreonyltyrosine, and cycloarginyltyrosine fall within the range defined above, more preferably the contents of cyclolysyltyrosine, cycloisoleucyltyrosine, cyclothreonyltyrosine, cycloaspartyltyrosine, cycloglutaminyltyrosine, and cycloarginyltyrosine fall within the range defined above, and even more preferably the contents of cyclolysyltyrosine, cycloisoleucyltyrosine, cyclothreonyltyrosine, cycloaspartyltyrosine, cycloasparaginyltyrosine, cycloglutaminyltyrosine, cycloarginyltyrosine, and cyclomethionyltyrosine fall within the range defined above, from the viewpoint of solubility in water.

The contents of the above-mentioned cyclic dipeptide or a salt thereof in Embodiment 3 are as mentioned above for each component, and a total content of the cyclic dipeptide or a salt thereof is preferably 0.008% by weight or more, more preferably 0.08% by weight or more, and even more preferably 0.16% by weight or more, and preferably 100% by weight or less, more preferably 80% by weight or less, and even more preferably 60% by weight or less.

In addition, a total content of cyclotyrosyltyrosine, cyclophenylalanyltyrosine, cycloleucyltyrosine, and salts thereof, each having a high action of inhibiting a xanthine oxidase, in Embodiment 3 is preferably 0.013% by weight or more, more preferably 0.13% by weight or more, and even more preferably 0.26% by weight or more, and preferably 23% by weight or less, more preferably 17.3% by weight or less, and even more preferably 11.5% by weight or less.

Among the cyclic dipeptides in Embodiment 3, a proportion of a total amount occupied by cyclotyrosyltyrosine, cyclophenylalanyltyrosine, cycloleucyltyrosine, and salts thereof, each having a high action of inhibiting a xanthine oxidase, is preferably 5% by weight or more, more preferably 10% by weight or more, and even more preferably 15% by weight or more, from the viewpoint of the effects of inhibiting a xanthine oxidase. In addition, although the upper limit is not particularly limited, the proportion of a total amount is preferably 90% by weight or less, and more preferably 60% by weight or less, from the viewpoint of solubilization property.

In addition, a weight ratio of cyclophenylalanyltyrosine to cyclotyrosyltyrosine [Cyclo(Tyr-Phe)/Cyclo(Tyr-Tyr)] is preferably from 95/5 to 35/65, more preferably from 90/10 to 40/60, and even more preferably from 90/10 to 50/50, from the viewpoint of the effects of inhibiting a xanthine oxidase.

Also, a weight ratio of cyclophenylalanyltyrosine to cyclotyrosylglycine [Cyclo(Phe-Tyr)/Cyclo(Tyr-Gly)] is preferably from 90/10 to 30/70, more preferably from 90/10 to 40/60, and even more preferably from 90/10 to 50/50, from the viewpoint of the effects of inhibiting a xanthine oxidase.

In addition, a total content of cyclolysyltyrosine, cycloarginyltyrosine, and salts thereof, each having a high solubility in water, in Embodiment 3 is preferably 0.02% by weight or more, more preferably 0.2% by weight or more, and even more preferably 0.4% by weight or more, and preferably 31 % by weight or less, more preferably 3.1% by weight or less, and even more preferably 1.55% by weight or less.

Among the cyclic dipeptides in Embodiment 3, a proportion of a total amount occupied by cyclolysyltyrosine, cycloarginyltyrosine, and salts thereof, each having a high solubility in water, is preferably % by weight or more, more preferably % by weight or more, and even more preferably % by weight or more, from the viewpoint of preparing a composition. In addition, although the upper limit is not particularly limited, the proportion of a total amount is preferably % by weight or less, and more preferably % by weight or less, from the viewpoint of solubilization property.

The composition of Embodiment 3 may be prepared by, for example, when preparing a known food composition, mixing a given amount of a cyclic dipeptide or a salt thereof mentioned above to the raw materials of the food composition, to prepare a food composition in accordance with a known method for producing a food composition. Alternatively, the composition may be prepared by adding the above cyclic dipeptide or a salt thereof to an already made known food composition so that it is contained in a given amount mentioned above. Here, a known food composition may be those originally containing the cyclic dipeptide or a salt thereof, or if the cyclic dipeptide of the present invention is contained in a given amount, the cyclic dipeptide can be properly blended to prepare the composition.

Specific examples of the composition of Embodiment 3 include retort-pouched foods, seasonings (e.g. sauce, soup, salad dressing, mayonnaise, cream), confectionaries and sweets (e.g. baked confectionaries such as bread, cakes, cookies, and biscuits, chewing gum, chocolate, candies), and desserts (e.g. jello, yogurt, ice cream). In addition, the form of the foodstuff is not particularly limited, and the form may be any of easily ingestible forms such as solids, powders, liquids, gel-like forms, and slurry forms. The composition may be blended with cuisines using foodstuff richly containing purines such as livers, and fishes and shells such as milts, shrimps, sardines, and bonitos, and other cuisines that match beer-taste beverages, such as dumplings, *nikujaga* (potatoes and meat boiled in soy sauce), pork *kakuni* (Japanese boiled pork), and hamburgers, and used.

The composition of the present invention can be ingested by an appropriate method in accordance with the form thereof. The method of ingestion is not particularly limited, so long as the cyclic dipeptide or a salt thereof of the present invention can migrate to the circulation blood. Here, the word ingestion or ingested as used herein is used to embrace all the embodiments of ingestion, internal ingestion (medication), or drinks.

The amount of ingestion of the composition of the present invention is appropriately set depending upon its form, method of administration, purposes of use, and age, body weight, symptoms of the suffering individuals or suffering animals to which the composition is to be ingested, and is not a certain level. For example, the effective human ingestion amount of the cyclic dipeptide or a salt thereof of the present invention according to the present invention is preferably 0.2 mg or more, more preferably 2 mg or more, and even more preferably 20 mg or more, and preferably 10 g or less, more preferably 5 g or less, and even more preferably 2 g or less, per day, per human body weight of 50 kg. In addition, the administration may be carried out in a single dose or divided in plural doses in one day, within the desired dose range. The administration period is also optional. Here, the effective human ingestion amount of the cyclic dipeptide or a salt thereof of the present invention refers to a total ingestion amount of the cyclic dipeptides or salts thereof showing effective effects in human, and the kinds of the cyclic dipeptides are not particularly limited.

The subjects to which the composition of the present invention is ingested herein refer to preferably human in need of lowering uric acid levels, which may be also livestock animals such as cattle (cows, bulls), horses, and goats, pet animals such as dogs, cats, and rabbits, or experimental animals such as mice, rats, guinea pigs, and monkeys.

EXAMPLES

The present invention will be more particularly described showing Examples, without intending to limit the scope of the present invention to the following Examples.

< Reagents >

As a cyclic dipeptide, one synthesized by KNC Laboratories Co., Ltd. was used. Each of xanthine, Sodium Carboxymethyl Cellulose (CMC-Na), tyrosine (Tyr), formic acid (Special Grade reagent for column chromatography), and methanol (used for high-performance liquid chromatography), manufactured by NACALAI TESQUE INC.; each of Allopurinol and potassium oxonate, manufactured by Wako Pure Chemical Industries, Ltd.; xanthine oxidase, manufactured by TOYOBO CO., LTD.; Xanthine Oxidase Assay Kit, manufactured by Cayman Chemical Company; L-Tyrosylglycine, manufactured by Santa Cruz Biotechnology Inc.; Glycyl-L-tyrosine, manufactured by Sigma-Aldrich; each of Tissue Protein Extraction Reagent (T-PER), Protease inhibitor cocktail kit, Pierce BCA protein assay kit, and polystyrene Black 96-well plate, manufactured by Thermo Scientific; heparin sodium, manufactured by MOCHIDA PHARMACEUTICAL CO., LTD.; Pravastatin sodium, manufactured by SIGMA; a soybean peptide (Hinute AM), manufactured by FUJI OIL, CO., LTD., were respectively used.

< Statistical Analysis >

In the subsequent Test Examples, data were indicated as mean $\pm$ standard error. As a statistical test, in Test Example 1 a Student's t-test was used, and in other Test Examples dispersion analysis was performed with a one-way ANOVA, and thereafter a multiple comparison test was carried out using Dunnet's test. In the results the denotation "*" means a significant difference $p < 0.05$, and the denotation "♯" means a significant difference $p < 0.1$. Here, all these analyses were performed using SPSS for Windows(registered trademark) release 17.0, manufactured by SPSS.

Test Example 1 (Studies on *in vitro* XO Inhibiting Action)

The *in vitro* xanthine oxidase (XO) inhibiting action was studied on 210 kinds of cyclic dipeptides, linear dipeptides Tyr-Gly and Gly-Tyr, and an amino acid Tyr.

Concretely, 75 $\mu$L of 4 U/L xanthine oxidase (XO) dissolved in a phosphate buffer (PBS), pH 7.5, was dispensed in each of the wells of a 96-well plate, and 5 $\mu$L of a sample solution was added thereto so that a peptide or amino acid had a given concentration based on the XO final concentration, and the liquid mixture was mixed for 5 minutes (a final concentration of a peptide or amino acid being from 50 to 500 $\mu$M). Thereafter, 20 $\mu$L of a 250 $\mu$M xanthine solution previously dissolved in the PBS was added thereto, and the absorbance after 30 minutes was measured using a spectrophotometer (Synegy HT, manufactured by Bio Tek). The XO inhibition rate (%) at each concentration was calculated by the following calculation formula, to obtain activities of inhibiting XO (IC$_{50}$ values). Table 1 shows activities of inhibiting a xanthine oxidase (IC$_{50}$ values) of 12 kinds of Tyr-containing cyclic dipeptides, Table 2 shows xanthine oxidase inhibition rates (%) of 8 kinds of Tyr-containing cyclic dipeptides at a concentration of 100 $\mu$M, and Table 3 shows activities of inhibiting a xanthine oxidase (IC$_{50}$ values) of 20 kinds of Phe-containing cyclic dipeptides. Here, as a positive control, a known uric acid level-lowering agent Allopurinol (IC$_{50}$ value: 12.5 $\mu$M) was utilized.

$$\%Inhibition = \left\{ \frac{(A - B) - (C - D)}{A - B} \right\} \times 100$$

wherein A: the absorbance of an enzyme-added, sample-non-added group at 295 nm;
B: the absorbance of an enzyme-non-added, sample-non-added group at 295 nm;
C: the absorbance of an enzyme-added, sample-added group at 295 nm; and
D: the absorbance of an enzyme-non-added, sample-added group at 295 nm.

[Table 1]

Table 1

| Activity of Inhibiting XO (Xanthine Oxidase) of Tyr(Tyrosine)-Containing Cyclic Dipeptides ||
|---|---|
| Compound | IC$_{50}$ ($\mu$M) |
| Cyclo(Ser-Tyr) | 188.9 $\pm$ 52.5 |
| Cyclo(Tyr-Gly) | 49.0 $\pm$ 3.0 |
| Cyclo(Tyr-Tyr) | 29.5 $\pm$ 2.9 |
| Cyclo(Phe-Tyr) | 22.2 $\pm$ 0.8 |
| Cyclo(Leu-Tyr) | 23.8 $\pm$ 5.3 |
| Cyclo(Val-Tyr) | 79.4 $\pm$ 16.1 |
| Cyclo(Ile-Tyr) | 86.7 $\pm$ 2.8 |
| Cyclo(Asp-Tyr) | 172 $\pm$ 13.5 |
| Cyclo(Asn-Tyr) | 113 $\pm$ 11.4 |
| Cyclo(Arg-Tyr) | 211 $\pm$ 36.2 |
| Cyclo(Met-Tyr) | 56.4 $\pm$ 2.9 |
| Cyclo(Tyr-Cys) | 77.2 $\pm$ 4.7 |

[Table 2]

Table 2

| Activity of Inhibiting XO (Xanthine Oxidase) of Tyr(Tyrosine)-Containing Cyclic Dipeptides ||
|---|---|
| Compound | Inhibition Rate (%) at Concentration of 100 $\mu$M |
| Cyclo(Trp-Tyr) | 19.1 |
| Cyclo(Pro-Tyr) | 2.3 |
| Cyclo(Lys-Tyr) | 25.7 |
| Cyclo(His-Tyr) | 3.2 |
| Cyclo(Ala-Tyr) | 8.3 |

(continued)

| Activity of Inhibiting XO (Xanthine Oxidase) of Tyr(Tyrosine)-Containing Cyclic Dipeptides | |
|---|---|
| Compound | Inhibition Rate (%) at Concentration of 100 $\mu$M |
| Cyclo(Glu-Tyr) | 0.4 |
| Cyclo(Thr-Tyr) | 2.6 |
| Cyclo(Gln-Tyr) | 42.6 |

[Table 3]

Table 3

| Activity of Inhibiting XO (Xanthine Oxidase) of Phe(Phenylalanine)-Containing Cyclic Dipeptides | |
|---|---|
| Compound | IC$_{50}$ ($\mu$M) |
| Cyclo(Trp-Phe) | >100 |
| Cyclo(Ser-Phe) | >300 |
| Cyclo(Pro-Phe) | >100 |
| Cyclo(Phe-Gly) | >100 |
| Cyclo(Phe-Tyr) | 22.2 $\pm$ 0.8 |
| Cyclo(Phe-Phe) | >100 |
| Cyclo(Leu-Phe) | >100 |
| Cyclo(Lys-Phe) | >300 |
| Cyclo(His-Phe) | >100 |
| Cyclo(Ala-Phe) | >100 |
| Cyclo(Glu-Phe) | >100 |
| Cyclo(Val-Phe) | 60.5 $\pm$ 2.4 |
| Cyclo(Ile-Phe) | >100 |
| Cyclo(Thr-Phe) | >300 |
| Cyclo(Asp-Phe) | >300 |
| Cyclo(Asn-Phe) | >300 |
| Cyclo(Gln-Phe) | >300 |
| Cyclo(Arg-Phe) | >300 |
| Cyclo(Met-Phe) | >100 |
| Cyclo(Phe-Cys) | 132.8 $\pm$ 25.2 |

It can be seen from Tables 1 and 2 that the Tyr-containing cyclic dipeptides have activities of inhibiting an XO, and from Table 3 that the activities of inhibiting an XO are attenuated when Tyr is replaced by Phe.

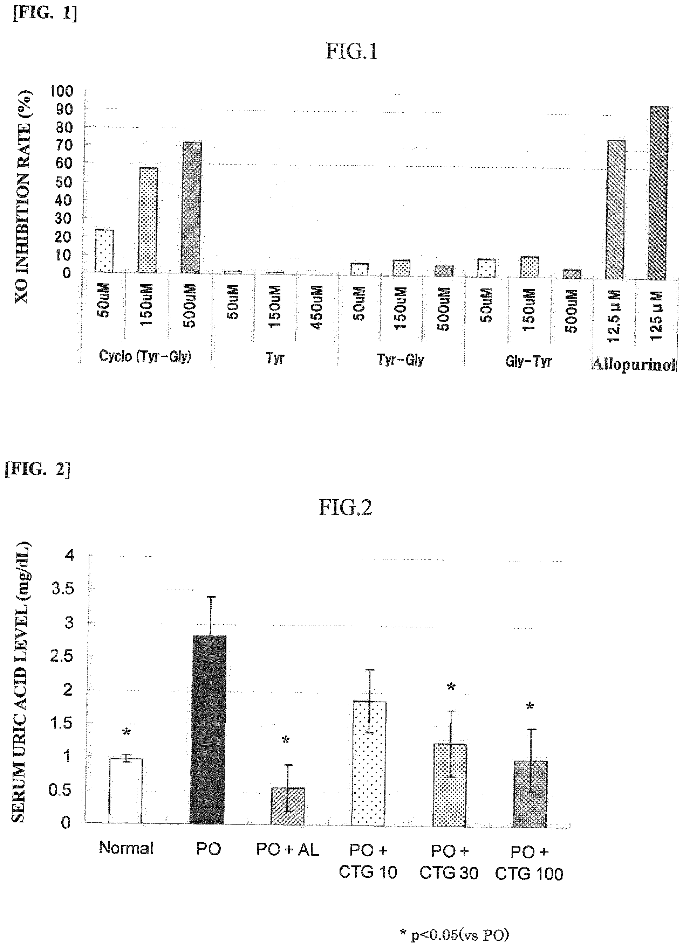

Also, the XO inhibition rates (%) were measured for Cyclo(Tyr-Gly), linear dipeptides Tyr-Gly and Gly-Tyr, and an amino acid Tyr in the same manner. The results are shown in FIG. 1.

It can be seen from FIG. 1 that the activity of inhibiting an XO is exhibited by cyclization, suggesting that it is important to be a cyclic dipeptide.

Test Example 2 Studies on Actions of Lowering Serum Uric Acid Levels for Hyperuricemic Mice)

The actions of lowering serum uric acid levels with cyclic dipeptides were studied in hyperuricemic model animals.

As to the animals used, male 7-week old BALBc mice were purchased from CLEA Japan, Inc., and subjected to an experiment after a one-week period of conditioning. The animals were bred in a breeding room with air-conditioning facilities (temperature: 23.5° ± 1.0°C, humidity: 55 ± 10 RH%, the number of ventilation: 12 to 15 times/hour, illuminated: from 7:00 to 19:00 each day). During the period of conditioning, the mice were allowed to take a commercially available feed (CE-2, manufactured by CLEA Japan, Inc.) and distilled water *ad libitum.*

Oxonic acid is a substance that raises a blood uric acid level by inhibiting a urikase, a metabolic enzyme for uric acid. In view of the above, potassium oxonate (PO) was administered, thereby generating hyperuricemic model animals (see, Planta Med 2009; 75:302-306). Studies were made on the following 6 groups:

1) normal group (Normal Group, without loading oxonic acid);
2) group administered with PO (PO Group, with loading oxonic acid);
3) group administered with PO + Allopurinol 1 mg/kg (PO + AL Group);
4) group administered with PO + Cyclo(Tyr-Gly) 10 mg/kg (PO + CTG 10 Group);
5) group administered with PO + Cyclo(Tyr-Gly) 30 mg/kg (PO + CTG 30 Group); and
6) group administered with PO + Cyclo(Tyr-Gly) 100 mg/kg (PO + CTG 100 Group),

wherein in each group, n = 6 to 8. In all the groups that include PO administration, PO previously suspended in a 0.5% aqueous CMC-Na solution at 250 mg/kg was administered, and Normal Group was administered with a 0.5% aqueous CMC-Na solution, each being administered intraperitoneally. Next, after one hour from the administration, the group administered with PO + Allopurinol was administered with 1 mg/kg Allopurinol, each of the groups administered with PO + Cyclo(Tyr-Gly) was administered with Cyclo(Tyr-Gly) at 10, 30, or 100 mg/kg, and Normal Group was administered with distilled water, each being administered orally. After 2 hours from the administration with PO, the blood was collected from the abdominal vena cava under anesthesia. After blood removal, the liver was harvested. The collected blood was allowed to stand at room temperature for 45 minutes, and centrifuged at 8,000 rpm for 10 minutes to recover sera. Here, the sera and the liver were preserved at -80°C until the measurements were carried out.

The serum uric acid level was measured using 7180 Clinical Analyzer (manufactured by Hitachi Technologies). The results are shown in FIG. 2.

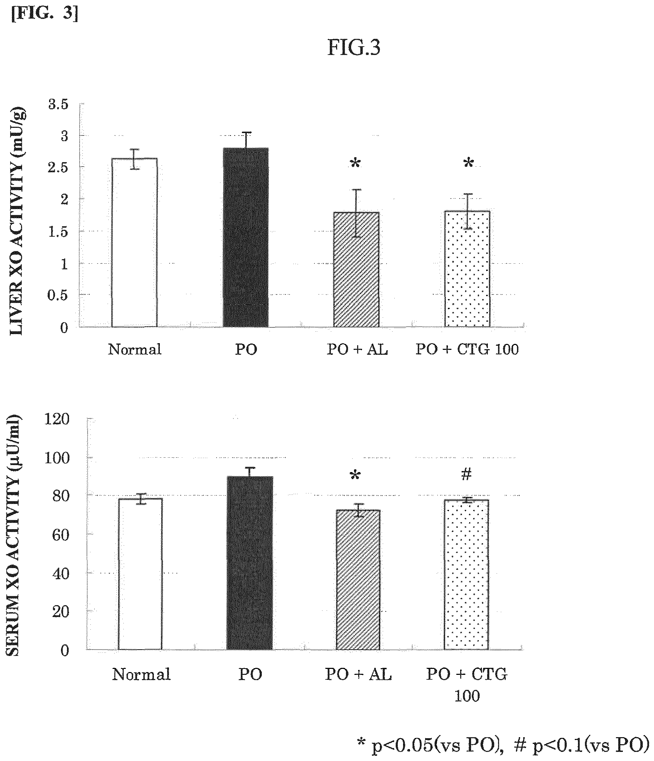

Next, the livers of mice were added to a mixture of an ice-cooled protease inhibitor cocktail and an EDTA-containing tissue protein extraction reagent (T-PER), homogenized, and then centrifuged at 15,000 rpm, at 4°C for 15 minutes, and the collected supernatant was subjected to an assay for a xanthine oxidase (XO) activity. The XO activities in the livers and the sera were assayed using a xanthine oxidase assay kit and a polystyrene Black 96-well plate. Concretely, XO standard samples, liver samples, or sera samples were added in an amount of 50 μL, and thereafter 50 μL of an analytical cocktail prepared by mixing an assay buffer, a Detector and HRP in a mass ratio of 98/1/1 was added thereto. The mixture was allowed to react at 37°C for 45 minutes while shading light, and a fluorescence was then measured at an excitation wavelength of from 520 to 550 nm and an emission wavelength of from 585 to 595 nm with a spectrophotometer (Synegy HT, manufactured by Bio Tek). The sera XO activities were indicated in units of mU/mL, and the XO activities in the liver were indicated in units of mU/mg, using the results of assaying a protein concentration with a Pierce BCA protein assay kit. The results are shown in FIG. 3.

It can be seen from FIG. 2 that Cyclo(Tyr-Gly) has an action of lowering a uric acid level in a dose-dependent manner, and that the group administered with Cyclo(Tyr-Gly) 30 or 100 mg/kg has a significant difference in effects on the PO Group, in the same manner as the group administered with Allopurinol. In addition, it can be seen from FIG. 3 that the xanthine oxidase activities in the liver and in the sera are inhibited in the group administered with Cyclo(Tyr-Gly) 100 mg/kg, in the same manner as the group administered with Allopurinol 1 mg/kg.

Test Example 3 (Studies on Action of Lowering Serum Uric Acid Levels of Soybean Peptide Heat-Treated Products for Hyperuricemic Mice)

The actions of lowering serum uric acid levels of soybean peptide heat-treated products were studied using hyperuricemic model animals, in the same manner as in Test Example 2. Concretely, the animals used were furnished in the same manner as in Test Example 2, and studies were made on the following 5 groups.

1) normal group (Normal Group, without loading oxonic acid);
2) group administered with PO (PO Group, with loading oxonic acid);
3) group administered with PO + Allopurinol 1 mg/kg (PO + AL Group);
4) group administered with PO + soybean peptide heat-treated product 2 g/kg; and
5) group administered with PO + 8 kinds of Tyr-containing cyclic dipeptide mixture 11 mg/kg,

wherein in each group, n = 6 to 7.

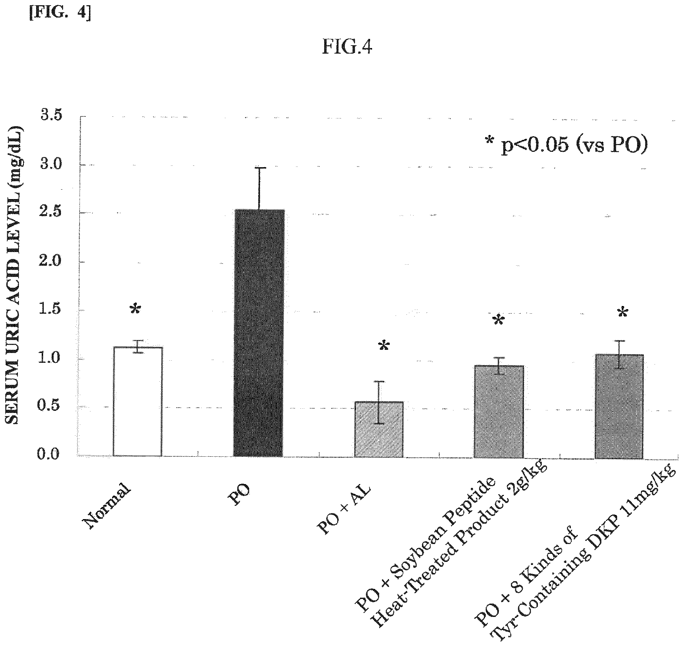

For each of the above-mentioned groups, the administration and the blood collection were carried out in the same manner as in Test Example 2 to measure a serum uric acid level. The results are shown in FIG. 4. Here, the mixture of the 8 kinds of cyclic dipeptides was prepared by using synthesized products of Cyclo(Ser-Tyr), Cyclo(Tyr-Gly), Cyclo(Tyr-Tyr), Cyclo(Phe-Tyr), Cyclo(Leu-Tyr), Cyclo(Val-Tyr), Cyclo(Ile-Tyr), and Cyclo(Asn-Tyr) in amounts so as to be contained in the soybean peptide heat-treated product and used.

Here, the heat-treated product of the soybean peptide obtained by dissolving a soybean peptide (Hinute AM, manufactured by FUJI OIL, CO., LTD.) in distilled water in a concentration of 200 mg/mL, heating to 132°C for 3 hours, and lyophilizing the heated mixture was used. The content of the cyclic dipeptide of the soybean peptide heat-treated product was measured according to LC-MS/MS under the following conditions. Here, Cyclo(Tyr-Cys) was undeterminable because the standard product was unstable in an aqueous solution. The results are shown in Table 4.

[LC-MS/MS Analytical Conditions]

| | |
|---|---|
| LC Apparatus | SHIMADZU UFLC XR |
| Column | Agilent technologies, Zorbax SB-AQ, 1.8 μm, 2.1 mm × 150 mm |
| Column Temperature | 40°C |
| Mobile Phase | Gradient Analyses of A: 0.1 % formic acid, and B: methanol |
| Flow Rate | 0.2 mL/min |
| Amount Injected | 2 μL |
| Detector | AB Sciex 4000 Q TRAP-Turbo Spray (ESI)-Scheduled MRM (multiple reaction monitoring) |

[Table 4]

Table 4

| Cyclic Dipeptide | Content ($\times 10^3$ppm) |
|---|---|
| Cyclo(Trp-Tyr) | 0.10 |
| Cyclo(Ser-Tyr) | 1.06 |
| Cyclo(Pro-Tyr) | 0.53 |
| Cyclo(Tyr-Gly) | 0.47 |
| Cyclo(Tyr-Tyr) | 0.18 |
| Cyclo(Phe-Tyr) | 0.74 |
| Cyclo(Leu-Tyr) | 0.92 |
| Cyclo(Lys-Tyr) | 1.25 |
| Cyclo(His-Tyr) | 0.32 |
| Cyclo(Ala-Tyr) | 0.52 |
| Cyclo(Glu-Tyr) | 0.22 |
| Cyclo(Val-Tyr) | 0.23 |
| Cyclo(Ile-Tyr) | 1.02 |
| Cyclo(Thr-Tyr) | 0.73 |
| Cyclo(Asp-Tyr) | 0.23 |
| Cyclo(Asn-Tyr) | 0.73 |
| Cyclo(Gln-Tyr) | 0.39 |
| Cyclo(Arg-Tyr) | 1.34 |
| Cyclo(Met-Tyr) | 0.17 |
| Cyclo(Tyr-Cys) | Undeterminable |

It can be seen from Table 4 and FIG. 4 that the soybean peptide heat-treated product contains a cyclic dipeptide of the present invention, and has an excellent action of lowering a blood uric acid level.

Test Example 4 (Studies on Effective Amount of Soybean Peptide Heat-Treated Products for Hyperuricemic Mice)

The actions of lowering serum uric acid levels depending on dose of soybean peptide heat-treated products were studied using hyperuricemic model animals, in the same manner as in Test Example 3. Concretely, the animals used were furnished in the same manner as in Test Example 2, and studies were made on the following 6 groups.

1) normal group (Normal Group, without loading oxonic acid);
2) group administered with PO (PO Group, with loading oxonic acid);
3) group administered with PO + Allopurinol 1 mg/kg (PO + AL Group);
4) group administered with PO + soybean peptide heat-treated product 200 mg/kg;
5) group administered with PO + soybean peptide heat-treated product 400 mg/kg; and
6) group administered with PO + soybean peptide heat-treated product 800 mg/kg,
wherein in each group, n = 5 to 6.

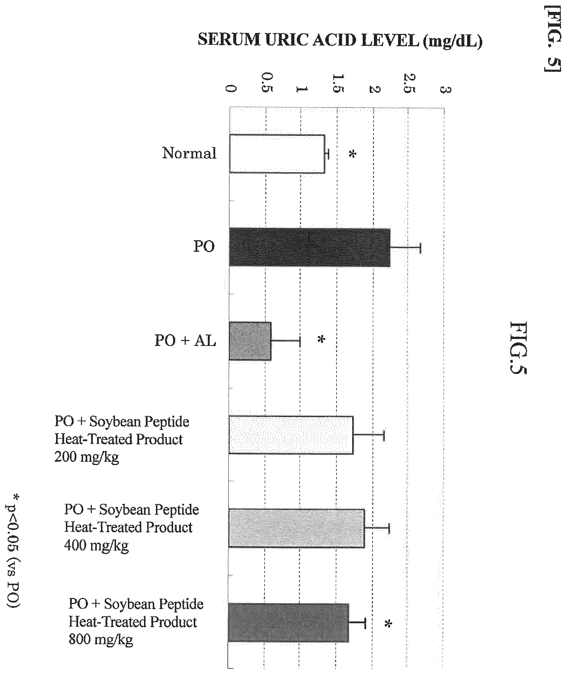

For each of the above-mentioned groups, the administration and the blood collection were carried out in the same manner as in Test Example 2 to measure a serum uric acid level. The results are shown in FIG. 5.

It can be seen from FIG. 5 that the soybean peptide heat-treated product is found to have a significant difference against the uric acid level of hyperuricemic model mice by administering the soybean peptide heat-treated product at 800 mg/kg, which is effective.

The cyclic dipeptide of the present invention can be prepared even from materials derived from foods, and the cyclic dipeptide is a material that is comfortably ingestible and has safety, so that the cyclic dipeptide has an advantage different from Allopurinol, which has some concerns in side effects. Although the cyclic dipeptide of the present invention might differ in the extent of actions depending upon the amounts used, the cyclic dipeptide is found to have actions of lowering a uric acid level that are equivalent or milder than that of Allopurinol depending upon the amounts used. The lowering of uric acid levels must be dealt over a long period of time together with dietary therapy, so that those having milder actions are said to be rather preferable, from the viewpoint of daily intake.

Test Example 5 (Studies on Solubility in Water)

The solubility in water was measured for cyclic dipeptides listed in Table 5.

Concretely, each compound was added to distilled water (manufactured by Otsuka Pharmaceutical Co., Ltd.) at an ambient temperature (25°C) so as to have a final concentration of 5 mM, and the mixture was stirred under ultrasonication for 30 minutes to dissolve. As a result, compounds that were completely dissolved by visual examination were expressed to have a solubility of "5 mM." Next, as to compounds that did not dissolve at a concentration of 5 mM, distilled water was added thereto so as to have a final concentration of 1 mM, and the mixture was stirred under ultrasonication for 30 minutes to dissolve. As a result, the dissolved compounds were expressed to have a solubility of "1 mM." Further, as to compounds that did not dissolve at a concentration of 1 mM, distilled water was added thereto so as to have a final concentration of 0.1 mM, and the mixture was stirred under ultrasonication for 30 minutes to confirm solubility. However, none of the compounds dissolved, so that distilled water was further added thereto so as to have a final concentration of 0.01 mM, and the mixture was stirred under ultrasonication for 30 minutes, but still no compounds dissolved. Such compounds were expressed to be "insoluble." The results are shown in Table 5.

[Table 5]

Table 5

| Cyclic Dipeptide | Solubility in Water |
| --- | --- |
| Cyclo(Trp-Tyr) | insoluble |
| Cyclo(Ser-Tyr) | insoluble |
| Cyclo(Pro-Tyr) | 1 mM |
| Cyclo(Tyr-Gly) | 1 mM |
| Cyclo(Tyr-Tyr) | insoluble |
| Cyclo(Phe-Tyr) | insoluble |

(continued)

| Cyclic Dipeptide | Solubility in Water |
|---|---|
| Cyclo(Leu-Tyr) | insoluble |
| Cyclo(Lys-Tyr) | 5 mM |
| Cyclo(His-Tyr) | 1 mM |
| Cyclo(Ala-Tyr) | insoluble |
| Cyclo(Glu-Tyr) | 5 mM |
| Cyclo(Val-Tyr) | insoluble |
| Cyclo(Ile-Tyr) | 1 mM |
| Cyclo(Thr-Tyr) | 1 mM |
| Cyclo(Asp-Tyr) | 5 mM |
| Cyclo(Asn-Tyr) | 1 mM |
| Cyclo(Gln-Tyr) | 1 mM |
| Cyclo(Arg-Tyr) | 5 mM |
| Cyclo(Met-Tyr) | 5 mM |

It is suggested from Table 5 that Cyclo(Pro-Tyr), Cyclo(Tyr-Gly), Cyclo(Lys-Tyr), Cyclo(His-Tyr), Cyclo(Glu-Tyr), Cyclo(Ile-Tyr), Cyclo(Thr-Tyr), Cyclo(Asp-Tyr), Cyclo(Asn-Tyr), Cyclo(Gln-Tyr), Cyclo(Arg-Tyr), and Cyclo(Met-Tyr) have high solubility in water, and among them, Cyclo(Lys-Tyr), Cyclo(Glu-Tyr), Cyclo(Asp-Tyr), Cyclo(Arg-Tyr), and Cyclo(Met-Tyr) have a solubility in water of 5 mM or more, so that they can be suitably used in the preparation of the composition.

Concrete formulations of the composition blended with a cyclic dipeptide or a salt thereof of the present invention will be exemplified hereinbelow. These compositions can be prepared by a known method.

(Production Examples) Production of Carbonated Beverages

Cyclic dipeptides synthesized by KNG Laboratories Co., Ltd. are used, each of raw materials in a blending proportion as listed in the following Table 6 is dissolved in water, a pH of the solution is then adjusted to 3.8 with phosphoric acid, and each of an antioxidant, a flavor, an acidulant, a sweetener, and a caramel pigment is added in a proper amount and stored for about 24 hours. During the storage, carbonic gas is added in a proper amount, and thereafter the mixture is subjected to the steps of filtration, bottle packing, and sterilization (heating at 65°C or higher for 10 minutes), to give a carbonated beverage.

[Table 6]

Table 6

| Name of Raw Material, Manufactured Product Name | Prod. Ex. (1) | Prod. Ex. (2) | Prod. Ex. (3) | Prod. Ex. (4) | Prod. Ex. (5) | Prod. Ex. (6) | Prod. Ex. (7) |
|---|---|---|---|---|---|---|---|
| | Amount (% by weight) | | | | | | |
| Cyclotyrosyltyrosine | $1.5 \times 10^{-7}$ ($1.5 \times 10^{-4}$ mg/100 mL) | | | | | | |
| Cyclophenylalanyltyrosine | $4 \times 10^{-7}$ ($4 \times 10^{-4}$ mg/100 mL) | | | | | | |
| Cycloleucyltyrosine | $5 \times 10^{-7}$ ($5 \times 10^{-4}$ mg/100 mL) | | | | | | |
| SOYAFIBE-S-LA200 (manufactured by FUJI OIL CO., LTD.) | 0.8 | - | - | 0.4 | 0.4 | 0.4 | 0.2 |
| PINE-UP (manufactured by Matsutani Chemical Industry Co., Ltd.) | 0 | 0.8 | - | 0.4 | 0.4 | - | 0.2 |

(continued)

|  | Prod. Ex. (1) | Prod. Ex. (2) | Prod. Ex. (3) | Prod. Ex. (4) | Prod. Ex. (5) | Prod. Ex. (6) | Prod. Ex. (7) |
|---|---|---|---|---|---|---|---|
| SM900 (manufactured by San-Ei Gen F. F. I.) | 0 | - | 0.8 | - | - | 0.4 | 0.2 |
| HINUTE AM (manufactured by FUJI OIL CO., LTD.) | 0.2 | 0.2 | 0.2 | 0.2 | 0.2 | 0.2 | 0.4 |

(Production Examples) Production Examples of Chocolates

Chocolate is produced using raw materials in blending proportions as listed in the following Table 7. The raw materials of Table 7 are introduced into a Hobart mixer, mixed in a moderate speed for 3 minutes, and further pressed with a roller and subjected to conching, to give a chocolate dough. This chocolate dough is subjected to a tempering treatment, and then charged into a mold, and cooled, to give chocolate of the present invention.
[Table 7]

Table 7

|  | Prod. Ex. (8) | Prod. Ex. (9) | Prod. Ex. (10) | Prod. Ex. (11) | Prod. Ex. (12) |
|---|---|---|---|---|---|
| Raw Materials | Blend (% by Weight) | | | | |
| Cyclotyrosyltyrosine | 0.10 | - | - | 0.05 | 0.05 |
| Cyclophenylalanyltyrosine | - | 0.10 | - | 0.05 | - |
| Cycloleucyltyrosine | - | - | 0.10 | - | 0.05 |
| Cacao mass | 18.7 | | | | |
| Cacao butter | 26.5 | | | | |
| Sugar | 34.7 | | | | |
| Whole Milk Powder | 19.5 | | | | |
| Lecithin | 0.5 | | | | |

(Production Examples) Production Examples of Beer-Taste Carbonated Beverages

Cyclic dipeptides that are synthesized by KNG Laboratories Co., Ltd. are used, raw materials in blending proportions as listed in the following Table 8 are each dissolved in water, and a pH of the solution is then adjusted with lactic acid to 3.7. An antioxidant, a flavor, an acidulant, a sweetener, a bittering agent, and a caramel pigment are each added thereto in a proper amount, and the liquid mixture is stored for about 24 hours. During the storage, carbonic gas is added in a proper amount, and the mixture is subsequently subjected to filtration, bottle packing, sterilization (heating at 65°C or higher for 10 minutes), to give a beer-taste carbonated beverage.
[Table 8]

Table 8

|  | Prod. Ex. (13) | Prod. Ex. (14) | Prod. Ex. (15) | Prod. Ex. (16) | Prod. Ex. (17) | Prod. Ex. (18) | Prod. Ex. (19) |
|---|---|---|---|---|---|---|---|
| Name of Raw Material, Manufactured Product Name | Amount (% by weight) | | | | | | |
| Cyclolysyltyrosine | $7 \times 10^{-7}$ ($7 \times 10^{-4}$ mg/100 mL) | | | | | | |
| Cycloisoleucyltyrosine | $5.5 \times 10^{-7}$ ($5.5 \times 10^{-4}$ mg/100 mL) | | | | | | |
| Cyclothreonyltyrosine | $4 \times 10^{-7}$ ($4 \times 10^{-4}$ mg/100 mL) | | | | | | |
| Cycloaspartyltyrosine | $1.5 \times 10^{-7}$ ($1.5 \times 10^{-4}$ mg/100 mL) | | | | | | |

(continued)

| | Prod. Ex. (13) | Prod. Ex. (14) | Prod. Ex. (15) | Prod. Ex. (16) | Prod. Ex. (17) | Prod. Ex. (18) | Prod. Ex. (19) |
|---|---|---|---|---|---|---|---|
| Cycloasparaginyltyrosine | $4 \times 10^{-7}$ ($4 \times 10^{-4}$ mg/100 mL) | | | | | | |
| Cycloglutaminyltyrosine | $2 \times 10^{-7}$ ($2 \times 10^{-4}$ mg/100 mL) | | | | | | |
| Cycloarginyltyrosine | $3.5 \times 10^{-7}$ ($3.5 \times 10^{-4}$ mg/100 mL) | | | | | | |
| Cyclomethionyltyrosine | $1.3 \times 10^{-7}$ ($1.3 \times 10^{-4}$ mg/100 mL) | | | | | | |
| SOYAFIBE-S-LA200 (manufactured by FUJI OIL CO., LTD.) | 0.8 | - | - | 0.4 | 0.4 | 0.4 | 0.2 |
| PINE-UP (manufactured by Matsutani Chemical Industry Co., Ltd.) | 0 | 0.8 | - | 0.4 | 0.4 | - | 0.2 |
| SM900 (manufactured by San-Ei Gen F. F. I.) | 0 | - | 0.8 | - | - | 0.2 | 0.2 |
| HINUTE AM (manufactured by FUJI OIL CO., LTD.) | 0.2 | 0.2 | 0.2 | 0.2 | 0.2 | 0.2 | 0.4 |

INDUSTRIAL APPLICABILITY

The cyclic dipeptide-containing composition of the present invention has an excellent an action of lowering a uric acid level, and the cyclic dipeptide-containing composition is useful in, for example, prevention or treatment of hyperuricemia, gout or the like.

**Claims**

1.   A cyclic dipeptide-containing composition, comprising one or more tyrosine-containing cyclic dipeptides or a salt thereof of the following (1) to (8) each in an amount satisfying ranges of:

(1) content of cyclolysyltyrosine or a salt thereof: $1.00 \times 10$ to $1.50 \times 10^5$ ppm,
(2) content of cycloisoleucyltyrosine or a salt thereof: $0.80 \times 10$ to $1.30 \times 10^5$ ppm,
(3) content of cyclothreonyltyrosine or a salt thereof: $0.50 \times 10$ to $0.90 \times 10^5$ ppm,
(4) content of cycloaspartyltyrosine or a salt thereof: $0.10 \times 10$ to $0.30 \times 10^5$ ppm,
(5) content of cycloasparaginyltyrosine or a salt thereof: $0.50 \times 10$ to $0.90 \times 10^5$ ppm,
(6) content of cycloglutaminyltyrosine or a salt thereof: $0.30 \times 10$ to $0.50 \times 10^5$ ppm,
(7) content of cycloarginyltyrosine or a salt thereof: $1.00 \times 10$ to $1.60 \times 10^5$ ppm, and
(8) content of cyclomethionyltyrosine or a salt thereof: $0.10 \times 10$ to $0.20 \times 10^5$ ppm.

2.   The composition according to claim 1, further comprising one or more tyrosine-containing cyclic dipeptides or a salt thereof of the following (9) to (19) each in an amount satisfying ranges of:

(9) content of cyclotryptophanyltyrosine or a salt thereof: $0.08 \times 10$ to $0.12 \times 10^5$ ppm,
(10) content of cycloseryltyrosine or a salt thereof: $0.80 \times 10$ to $1.30 \times 10^5$ ppm,
(11) content of cycloprolyltyrosine or a salt thereof: $0.40 \times 10$ to $0.70 \times 10^5$ ppm,
(12) content of cyclotyrosylglycine or a salt thereof: $0.30 \times 10$ to $0.60 \times 10^5$ ppm,
(13) content of cyclotyrosyltyrosine or a salt thereof: $0.10 \times 10$ to $0.30 \times 10^5$ ppm,
(14) content of cyclophenylalanyltyrosine or a salt thereof: $0.50 \times 10$ to $0.90 \times 10^5$ ppm,
(15) content of cycloleucyltyrosine or a salt thereof: $0.70 \times 10$ to $1.10 \times 10^5$ ppm,
(16) content of cyclohistidyltyrosine or a salt thereof: $0.20 \times 10$ to $0.40 \times 10^5$ ppm,
(17) content of cycloalanyltyrosine or a salt thereof: $0.40 \times 10$ to $0.70 \times 10^5$ ppm,
(18) content of cycloglutamyltyrosine or a salt thereof: $0.10 \times 10$ to $0.30 \times 10^5$ ppm, and
(19) content of cyclovalyltyrosine or a salt thereof: $0.10 \times 10$ to $0.30 \times 10^5$ ppm.

3. The composition according to claim 1 or 2, wherein a total amount of the tyrosine-containing cyclic dipeptide or a salt thereof is from $8.0 \times 10$ to $1.0 \times 10^6$ ppm.

4. A cyclic dipeptide-containing composition, comprising one or more tyrosine-containing cyclic dipeptides or a salt thereof of the following (1) to (8) each in an amount satisfying ranges of:

    (1) content of cyclolysyltyrosine or a salt thereof: $5.0 \times 10^{-4}$ to $9.0 \times 10$ mg/100 mL,
    (2) content of cycloisoleucyltyrosine or a salt thereof: $4.0 \times 10^{-4}$ to $7.0 \times 10$ mg/100 mL,
    (3) content of cyclothreonyltyrosine or a salt thereof: $3.0 \times 10^{-4}$ to $5.0 \times 10$ mg/100 mL,
    (4) content of cycloaspartyltyrosine or a salt thereof: $1.0 \times 10^{-4}$ to $2.0 \times 10$ mg/100 mL,
    (5) content of cycloasparaginyltyrosine or a salt thereof: $3.0 \times 10^{-4}$ to $5.0 \times 10$ mg/100 mL,
    (6) content of cycloglutaminyltyrosine or a salt thereof: $1.0 \times 10^{-4}$ to $3.0 \times 10$ mg/100 mL,
    (7) content of cycloarginyltyrosine or a salt thereof: $6.0 \times 10^{-4}$ to $1.0 \times 10^2$ mg/100 mL, and
    (8) content of cyclomethionyltyrosine or a salt thereof: $0.7 \times 10^{-4}$ to $2.0 \times 10$ mg/100 mL.

5. The composition according to claim 4, further comprising one or more tyrosine-containing cyclic dipeptides or a salt thereof of the following (9) to (19) each in an amount satisfying ranges of:

    (9) content of cyclotryptophanyltyrosine or a salt thereof: $0.4 \times 10^{-4}$ to $0.7 \times 10$ mg/100 mL,
    (10) content of cycloseryltyrosine or a salt thereof: $4.0 \times 10^{-4}$ to $8.0 \times 10$ mg/100 mL,
    (11) content of cycloprolyltyrosine or a salt thereof: $2.0 \times 10^{-4}$ to $4.0 \times 10$ mg/100 mL,
    (12) content of cyclotyrosylglycine or a salt thereof: $2.0 \times 10^{-4}$ to $4.0 \times 10$ mg/ 100 mL,
    (13) content of cyclotyrosyltyrosine or a salt thereof: $0.8 \times 10^{-4}$ to $2.0 \times 10$ mg/100 mL,
    (14) content of cyclophenylalanyltyrosine or a salt thereof: $3.0 \times 10^{-4}$ to $5.0 \times 10$ mg/ 100 mL,
    (15) content of cycloleucyltyrosine or a salt thereof: $4.0 \times 10^{-4}$ to $7.0 \times 10$ mg/100 mL,
    (16) content of cyclohistidyltyrosine or a salt thereof: $1.0 \times 10^{-4}$ to $3.0 \times 10$ mg/100 mL,
    (17) content of cycloalanyltyrosine or a salt thereof: $2.0 \times 10^{-4}$ to $4.0 \times 10$ mg/ 100 mL,
    (18) content of cycloglutamyltyrosine or a salt thereof: $1.0 \times 10^{-4}$ to $2.0 \times 10$ mg/100 mL, and
    (19) content of cyclovalyltyrosine or a salt thereof: $1.0 \times 10^{-4}$ to $2.0 \times 10$ mg/100 mL.

6. The composition according to claim 4 or 5, wherein a total amount of the tyrosine-containing cyclic dipeptide or a salt thereof is from $5.0 \times 10^{-3}$ to $8.0 \times 10^2$ mg/100 mL.

7. A cyclic dipeptide-containing composition, comprising one or more tyrosine-containing cyclic dipeptides or a salt thereof of the following (1) to (8) each in an amount satisfying ranges of:

    (1) content of cyclolysyltyrosine or a salt thereof: 0.01 to 15% by weight,
    (2) content of cycloisoleucyltyrosine or a salt thereof: 0.008 to 13% by weight,
    (3) content of cyclothreonyltyrosine or a salt thereof: 0.005 to 9% by weight,
    (4) content of cycloaspartyltyrosine or a salt thereof: 0.001 to 3% by weight,
    (5) content of cycloasparaginyltyrosine or a salt thereof: 0.005 to 9% by weight,
    (6) content of cycloglutaminyltyrosine or a salt thereof: 0.003 to 5% by weight,
    (7) content of cycloarginyltyrosine or a salt thereof: 0.01 to 16% by weight, and
    (8) content of cyclomethionyltyrosine or a salt thereof: 0.001 to 2% by weight.

8. The composition according to claim 7, further comprising one or more tyrosine-containing cyclic dipeptides or a salt thereof of the following (9) to (19) each in an amount satisfying ranges of: (

    9) content of cyclotryptophanyltyrosine or a salt thereof: 0.0008 to 1.2% by weight,
    (10) content of cycloseryltyrosine or a salt thereof: 0.008 to 13% by weight,
    (11) content of cycloprolyltyrosine or a salt thereof: 0.004 to 7% by weight,
    (12) content of cyclotyrosylglycine or a salt thereof: 0.003 to 6% by weight,
    (13) content of cyclotyrosyltyrosine or a salt thereof: 0.001 to 3% by weight,
    (14) content of cyclophenylalanyltyrosine or a salt thereof: 0.005 to 9% by weight,
    (15) content of cycloleucyltyrosine or a salt thereof: 0.007 to 11% by weight,
    (16) content of cyclohistidyltyrosine or a salt thereof: 0.002 to 4% by weight,
    (17) content of cycloalanyltyrosine or a salt thereof: 0.004 to 7% by weight,
    (18) content of cycloglutamyltyrosine or a salt thereof: 0.001 to 3% by weight, and

(19) content of cyclovalyltyrosine or a salt thereof: 0.001 to 3% by weight.

**9.** The composition according to claim 7 or 8, wherein a total amount of the tyrosine-containing cyclic dipeptide or a salt thereof is from 0.008 to 100% by weight.

**10.** The composition according to any one of claims 1 to 9, wherein the composition comprising the tyrosine-containing cyclic dipeptide is a processed product obtained by subjecting a solution comprising a soybean peptide to a heat treatment.

[FIG. 1]

# FIG.1

[FIG. 2]

# FIG.2

* p<0.05(vs PO)

[FIG. 3]

FIG.3

* p<0.05(vs PO), # p<0.1(vs PO)

[FIG. 4]

FIG.4

[FIG. 5]

FIG.5

* p<0.05 (vs PO)

**INTERNATIONAL SEARCH REPORT**

| International application No. |
| --- |
| PCT/JP2015/054015 |

**A. CLASSIFICATION OF SUBJECT MATTER**
*C07K5/12*(2006.01)i, *A23G1/00*(2006.01)i, *A23G1/30*(2006.01)i, *A23L1/305* (2006.01)i, *A23L2/00*(2006.01)i, *C07D241/08*(2006.01)i, *C07K5/06*(2006.01)i

According to International Patent Classification (IPC) or to both national classification and IPC

**B. FIELDS SEARCHED**

Minimum documentation searched (classification system followed by classification symbols)
C07K5/12, A23G1/00, A23G1/30, A23L1/305, A23L2/00, C07D241/08, C07K5/06

Documentation searched other than minimum documentation to the extent that such documents are included in the fields searched
Jitsuyo Shinan Koho         1922–1996    Jitsuyo Shinan Toroku Koho    1996–2015
Kokai Jitsuyo Shinan Koho   1971–2015    Toroku Jitsuyo Shinan Koho    1994–2015

Electronic data base consulted during the international search (name of data base and, where practicable, search terms used)
CAplus/REGISTRY/MEDLINE/EMBASE/BIOSIS/WPIDS/FSTA/FROSTI(STN),
JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| X | XIE, Z. et al., Organo- and hydrogels derived from cyclo(L-Tyr-L-Lys) and its ε-amino derivatives, Soft Matter, 2009.02.13, Vol.5, No.7, p.1474-1482 | 1,3,4,6,7,9 |
| X | QI, S.H. et al., Antibacterial and antilarval compounds from marine bacterium Pseudomonas rhizosphaerae, Annals of Microbiology, 2009.06, Vol.59, No.2, p.229-233 | 1,3,4,6,7,9 |
| X | ZENG, Y. et al., Synthesis of a small library of diketopiperazines as potential inhibitors of calpain, Bioorganic & Medicinal Chemistry Letters, 2005.05.17, Vol.15, No.12, p.3034-3038 | 1,3,4,6,7,9 |

| ☒ | Further documents are listed in the continuation of Box C. | ☐ | See patent family annex. |
| --- | --- | --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 08 May 2015 (08.05.15) | 19 May 2015 (19.05.15) |

| Name and mailing address of the ISA/ | Authorized officer |
| --- | --- |
| Japan Patent Office 3-4-3,Kasumigaseki,Chiyoda-ku, Tokyo 100-8915,Japan | |
| | Telephone No. |

Form PCT/ISA/210 (second sheet) (July 2009)

C (Continuation).    DOCUMENTS CONSIDERED TO BE RELEVANT

| Category* | Citation of document, with indication, where appropriate, of the relevant passages | Relevant to claim No. |
| --- | --- | --- |
| X | MAZUR, R.H. et al., Structure-taste relationships of some dipeptides, Journal of the American Chemical Society, 1969.05.07, Vol.91, No.10, p.2684-2691 | 1,3,4,6,7,9 |
| X | FONVIELLE, M. et al., Substrate and reaction specificity of Mycobacterium tuberculosis cytochrome P450 CYP121: insights from biochemical studies and crystal structures, The Journal of Biological Chemistry, 2013.04.25, Vol.288, No.24, p.17347-17359 | 1,3,4,6,7,9 |
| X | SASAKI, Y. et al., Studies on Analgesic Oligopeptides. II. Structure-Activity Relationship among Thirty Analogs of a Cyclic Dipeptide, Cyclo (-Tyr-Arg-), Chemical & pharmaceutical bulletin, 1982.12.25, Vol.30, No.12, p.4435-4443 | 1-9 |
| X | WO 1996/000391 A1  (AFFYMAX TECHNOLOGIES N.V.), 04 January 1996 (04.01.1996), entire text & US 5525734 A        & US 5525735 A & US 5817751 A        & WO 1995/035278 A1 | 1,3,4,6,7,9 |
| Y | JP 2003-252896 A  (President of Toyohashi University of Technology), 10 September 2003 (10.09.2003), entire text (Family: none) | 1-10 |
| Y | JP 5456876 B1  (Jellice Co., Ltd.), 17 January 2014 (17.01.2014), entire text (Family: none) | 1-10 |
| X<br>Y | PRASAD, C. et al., Could dietary proteins serve as cyclo(His-Pro) precursors?, Neuropeptides, 1991.05, Vol.19, No.1, p.17-21 | 1-10<br>1-10 |
| Y | CHMIELEWSKA, I. et al., Diketopiperazines, the non-assimilable components of pancreatic hydrolysates of protein for infusion, Acta Biochimica Polonica, 1967, Vol.14, No.4, p.409-414 | 1-10 |
| Y | ERIKSEN, S.A. et al., NON-VOLATILE NITROGEN COMPOUNDS IN HYDROLYZED VEGETABLE PROTEIN, Cereals for Food and Beverages, Recent Progress in Cereal Chemistry and Technology, 1980, p.395-408 | 1-10 |

Form PCT/ISA/210 (continuation of second sheet) (July 2009)

**INTERNATIONAL SEARCH REPORT**

| International application No. |
| --- |
| PCT/JP2015/054015 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | JP 2013-053115 A (Josho Gakuen Educational Foundation), 21 March 2013 (21.03.2013), entire text (Family: none) | 1-10 |
| A | NONGONIERMA, A.B. et al., Tryptophan-containing milk protein-derived dipeptides inhibit xanthine oxidase, Peptides, 2012.08.13, Vol.37, No.2, p.263-272 | 1-10 |
| A | JP 2006-111541 A (Toyo Shinyaku Co., Ltd.), 27 April 2006 (27.04.2006), entire text (Family: none) | 1-10 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012517998 A
- JP 2003252896 A

**Non-patent literature cited in the description**

- *Peptides,* 1995, vol. 16 (1), 151-164
- *Biosciences and Industries,* 2002, vol. 60 (7), 454-457
- *Chemical Reviews,* 2012, vol. 112, 3641-3716
- *J. Peptide Sci.,* 2004, vol. 10, 737-737
- *Planta Med,* 2009, vol. 75, 302-306